# FASCICULE DE BREVET EUROPEEN

(11) **EP 4 295 881 B1**
(45) Date de publication et mention de la délivrance du brevet: **23.07.2025**
(21) Numéro de dépôt: 23167338.5
(22) Date de dépôt: 11.04.2023
(51) Int. Cl.: A61M 16/00, A61M 16/08, A61M 16/10, A61M 16/20, A61M 16/22, A62B 7/08, B01D 53/00, C01B 13/02, B01D 53/047, B01D 53/22, B01D 53/32

(54) **INSTALLATION DE FOURNITURE D'OXYGÈNE À UN PATIENT INCLUANT UN MODULE DE SÉPARATION ÉLECTROCHIMIQUE À MEMBRANE CÉRAMIQUE**
ANLAGE ZUR SAUERSTOFFVERSORGUNG EINES PATIENTEN MIT EINEM ELEKTROCHEMISCHEN TRENNMODUL MIT KERAMISCHER MEMBRAN
PATIENT OXYGEN SUPPLY FACILITY INCLUDING CERAMIC MEMBRANE ELECTROCHEMICAL SEPARATION MODULE

(30) Priorité: 24.06.2022 FR 2206313
(43) Date de publication de la demande: 27.12.2023
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: BOULANGER, Thierry, 75321 Paris Cedex 07 (FR); BRANDANI, Federico, 78350 Jouy en Josas (FR); PULLUMBI, Pluton, 78350 Jouy en Josas (FR)
(74) Mandataire: Air Liquide

(56) Documents cités:
- WO-A1-98/22173
- US-A- 5 352 272
- US-A1- 2019 329 076

## Description

La présente invention concerne une installation de fourniture de gaz respiratoire, tel de l'oxygène pur ou quasiment pur, à un individu, i.e. un être humain, typiquement un patient en état hypoxémique, incluant un module de séparation électrochimique à membrane céramique. Une telle installation peut être utilisée pour traiter notamment une personne en état hypoxémique infectée par un coronavirus, tel le Covid-19 ou analogue, que ce soit un adulte, notamment une personne âgée, un adolescent ou enfant.

L'administration d'oxygène (O₂) gazeux sert à corriger une situation hypoxémique, c'est-à-dire lorsque la saturation d'oxyhémoglobine dans le sang ou « saturation en oxygène » devient ou est inférieure à une valeur normale, i.e. hors de la plage 95-100%, en particulier inférieure à 90%, chez un individu humain, i.e. un patient dans une population de patients de type nouveaux nés, enfants ou adultes, souffrant d'une pathologie respiratoire, par exemple une Bronchopneumopathie Chronique Obstructive (BPCO) ou un Syndrome de Détresse Respiratoire Aigüe (SDRA), notamment dans le cadre de la pandémie lié au coronavirus Covid-19.

A l'hôpital, l'oxygène provient d'un (ou plusieurs) réservoir de grande capacité (i.e. stockage de plusieurs milliers de litres), notamment contenant de l'oxygène sous forme liquide (LOX), qui est d'abord vaporisé, puis acheminé et enfin délivré par une ou des prises de distribution de gaz murales agencées dans les pièces de l'hôpital ou analogue, notamment les chambres, les salles de soins.... Les prises sont connectées fluidiquement au réseau de canalisations d'oxygène de l'hôpital alimenté par le stockage de grande capacité. Une telle installation permet, en temps normal, d'assurer un apport continu d'O₂ gazeux aux patients. Le réservoir de grande capacité doit être régulièrement réapprovisionné en LOX, typiquement par camion-citerne.

Selon la criticité de l'état du patient, des débits d'oxygène pouvant être élevés, typiquement pouvant atteindre 15 L/min, voire plus. Le débit d'oxygène souhaité est généralement réglé au moyen d'un débitmètre raccordé fluidiquement à la prise murale fournissant l'oxygène, puis acheminé au patient en ayant besoin, via un conduit d'acheminement de gaz raccordé fluidiquement à la sortie du débitmètre et alimentant une interface respiratoire, tel un masque respiratoire naso-buccal (i.e. facial). Ceci permet d'obtenir une fraction inspirée en oxygène (FiO₂) par le patient qui est supérieure à 90% et, en général, proche de 100%.

Or, la pandémie liée au coronavirus COVID-19 a démultiplié le nombre de patients hypoxémiques devant être traités avec de hautes concentrations d'O₂ pour rétablir leur saturation en oxygène normale, ce qui a engendré de nombreux problèmes d'approvisionnement en oxygène dans les pays développés et même rendu la tâche quasi-impossible dans les pays moins avancés, dans lesquels l'accès à l'O₂ est plus difficile, que ce soit pour des raisons de production, de logistique ou autre, entraînant une crise sanitaire inédite pour la population, en particulier pour les malades qui ont été privés ou limités en oxygène.

FR2106772 propose de réduire la consommation d'oxygène en éliminant le CO₂ des gaz expirés en utilisant des cartouches d'adsorbant. Le gaz épuré riche en oxygène peut être réinhalé ensuite par le patient. L'adsorbant est périodiquement régénéré par un flux d'air ambient. Toutefois, ce type de système peut s'avérer insuffisant lorsque des quantités d'oxygène importantes sont requises alors que les sources d'oxygène disponibles sont limitées ou subissent des contraintes, notamment d'encombrement, comme dans les maisons de retraites et/ou les zones rurales éloignées ou difficilement accessibles, ou encore dans le cadre d'une évacuation sanitaire de plusieurs patients par avion, train ou autre. De plus, la régénération par de l'air ambiant est incomplète, ce qui impacte la capacité d'adsorption qui se réduit au fil du temps, et nécessite maintenance fréquente.

Alternativement, il est connu, notamment de US-A-4,859,217, EP-A-0785020 et EP-A-1157731, de pouvoir utiliser une installation d'adsorption par modulation de pression de type PSA (pour Pressure Swing Adsorption) pour séparer, directement « sur site », l'air ambient et produire de l'O₂ gazeux à environ 90% de pureté. Cependant, une installation PSA de ce type ne permet pas de délivrer des débits supérieurs à 10 L/min et est par ailleurs lourde et encombrante. Il n'est par ailleurs pas possible de substituer une installation PSA a la source d'O₂ utilisée dans FR2106772 car les PSA génèrent une mixture de gaz riche en azote et argon ne pouvant être épurés et s'accumulant ainsi au détriment du niveau d'oxygène.

On connait par ailleurs WO98/22173 qui décrit une installation d'anesthésie comprenant une source de gaz pour fournir un gaz anesthésique contenant de l'oxygène, une ligne principale d'acheminement du gaz jusqu'à une interface respiratoire, une ligne de récupération de gaz pour amener les gaz expirés par le patient à un système de purification de gaz et une ligne de recyclage de gaz.

On connait également US 2019/329076 A1 qui décrit un système de régénération d'absorbeurs.

Par ailleurs, US2006/062707 propose un dispositif de génération d'oxygène à membrane céramique permettant de produire de l'oxygène à partir d'air ambiant.

Au vu de cela, il est nécessaire de proposer une installation de fourniture d'O₂ améliorée permettant un recyclage efficace des gaz expirés avec purification continue du CO₂, une régénération améliorée des adsorbeurs ou cartouches d'adsorption et une production in-situ simultanée d'oxygène à partir d'air ambiant, tout en limitant la consommation électrique et qui soit de conception simple.

Selon l'invention, il est proposé une installation de fourniture de gaz respiratoire à un utilisateur, i.e. un patient, en particulier de l'oxygène, comprenant :
- une source de gaz pour fournir un gaz respiratoire contenant de l'oxygène,
- une ligne principale d'acheminement de gaz reliant fluidiquement la source de gaz à une interface respiratoire, configurée pour administrer le gaz respiratoire contenant de l'oxygène à l'utilisateur, lors de chaque phase inspiratoire dudit utilisateur,
- une ligne de récupération de gaz configurée pour récupérer et acheminer au moins une partie du mélange gazeux CO₂/O₂ expiré par l'utilisateur et se retrouvant dans l'interface respiratoire, lors de chaque phase expiratoire de l'utilisateur, ladite ligne de récupération de gaz reliant fluidiquement l'interface respiratoire à un système de purification de gaz comprenant des adsorbeurs agencés en parallèle contenant chacun au moins un adsorbant, et
- une ligne de recyclage de gaz reliant fluidiquement le système de purification de gaz à la ligne principale d'acheminement de gaz.

Selon l'invention, la source de gaz de l'installation de fourniture de gaz respiratoire comprend une unité de génération d'oxygène, ladite unité de génération d'oxygène comprenant :
- au moins un module de séparation électrochimique,
- une première ligne de gaz pour alimenter ledit audit au moins un module de séparation électrochimique avec de l'air,
- des moyens de chauffage de gaz agencés sur la première ligne de gaz interne, en amont dudit au moins un module de séparation électrochimique et
- une seconde ligne de gaz reliant fluidiquement ledit au moins un module de séparation électrochimique au système de purification de gaz, pour convoyer un flux de gaz-déchet jusqu'au système de purification de gaz pour régénérer au ledit moins un adsorbant contenu dans au moins un desdits adsorbeurs.

En effet, utiliser le gaz-déchet pour régénérer le ou les adsorbants contenus dans les adsorbeurs est particulièrement avantageux car il est à haute température, par exemple de l'ordre de 300°C, ce qui permet la régénération du ou des adsorbants.

Selon le mode de réalisation considéré, l'installation de fourniture de gaz selon l'invention peut comprendre l'une ou plusieurs des caractéristiques suivantes :
- la source de gaz, i.e. l'unité de génération d'oxygène, est configurée pour fournir un gaz respiratoire contenant au moins 95% vol. d'oxygène, en particulier de l'oxygène pur ou quasiment pur, c'est-à-dire ayant une pureté d'au moins 98% vol. environ, de préférence d'au moins 99% vol. environ, de préférence encore d'au moins 99.5% vol.
- l'interface respiratoire est configurée pour administrer un gaz respiratoire à l'utilisateur contenant au moins 98% vol. d'oxygène, typiquement de l'oxygène quasiment pur, c'est-à-dire ayant une pureté d'au moins 99% vol. environ, lors de chaque phase inspiratoire de l'utilisateur,

- la ligne principale d'acheminement de gaz est configurée pour acheminer le gaz respiratoire contenant au moins 95% vol. d'oxygène de la source de gaz à l'interface respiratoire.
- la ligne de récupération de gaz est configurée pour récupérer et acheminer au moins une partie du mélange gazeux CO₂/O₂ expiré par l'utilisateur, i.e. se retrouvant dans l'interface respiratoire, lors de chaque phase expiratoire de l'utilisateur,
- chaque adsorbeur du système de purification de gaz est configuré pour fonctionner selon des cycles d'adsorption/régénération.
- le système de purification de gaz est configuré pour être alimenté en mélange gazeux CO₂/O₂ expiré par la ligne de récupération de gaz.
- le système de purification de gaz est en outre configuré pour éliminer au moins une partie du CO₂ contenu dans le mélange gazeux CO₂/O₂ expiré et obtenir un gaz purifié (i.e. gaz recyclé) contenant majoritairement de l'O₂, typiquement ayant une pureté d'au moins 95% vol. environ.
- la ligne de recyclage de gaz reliant fluidiquement le système de purification de gaz à la ligne principale d'acheminement de gaz est configurée pour acheminer au moins une partie du gaz purifié contenant majoritairement de l'O₂ provenant du système de purification de gaz et le réinjecter dans la ligne principale d'acheminement de gaz, de préférence via un réservoir-tampon.
   - le système de purification de gaz est ou comprend un dispositif de purification de gaz.
   - le système de purification de gaz comprend au moins trois adsorbeurs (aussi appelés cartouches) agencés en parallèle, typiquement de 3 à 5 adsorbeurs, de préférence 3 adsorbeurs.
   - la ligne principale d'acheminement de gaz comprend un réservoir-tampon et la ligne de recyclage de gaz est raccordée fluidiquement au réservoir-tampon.
   - la ligne de recyclage de gaz est raccordée fluidiquement au réservoir-tampon pour l'alimenter en gaz purifié (i.e. gaz recyclé) provenant du système de purification de gaz, de préférence un gaz recyclé contenant au moins 95% vol. d'oxygène.
   - le réservoir-tampon est agencé sur la ligne principale d'acheminement de gaz de sorte que le gaz véhiculé par la ligne principale d'acheminement de gaz successivement pénètre et ressorte du réservoir-tampon.
   - la ligne principale d'acheminement de gaz véhicule de l'oxygène de haute pureté, i.e. ayant une pureté d'au moins 99% vol., de préférence au moins 99.5% vol.
   - le système de purification de gaz est relié fluidiquement à la première ligne de gaz de l'unité de génération d'oxygène pour fournir de l'air à ladite première ligne de gaz.
   - le système de purification de gaz est relié fluidiquement à la première ligne de gaz de l'unité de génération d'oxygène via un premier conduit d'interconnexion.
   - le système de purification de gaz comprend un circuit d'air pour acheminer de l'air fourni à la première ligne de gaz de l'unité de génération d'oxygène.
   - le circuit d'air véhicule l'air au sein du système de purification de gaz entre un conduit d'admission d'air et un conduit d'extraction d'air.
   - le circuit d'air du système de purification de gaz comprend des passages ou conduits d'air et au moins un adsorbeur,
   - le circuit d'air du système de purification de gaz comprend en outre au moins une vanne de contrôle, en particulier une ou des vannes rotatives.
   - au moins un adsorbeur est agencé sur le circuit d'air entre le conduit d'admission d'air et le conduit d'extraction d'air.
   - le conduit d'admission d'air comprend un port d'entrée d'air et le conduit d'extraction d'air comprend un port de sortie d'air.
   - au moins une vanne de contrôle est agencée sur le circuit d'air entre le conduit d'admission d'air et au moins un adsorbeur.
   - au moins une vanne de contrôle est agencée sur le circuit d'air entre au moins un adsorbeur et le conduit d'extraction d'air.
   - le système de purification de gaz comprend en outre un circuit de régénération pour acheminer le gaz-déchet fourni par l'unité de génération d'oxygène.
   - le circuit de régénération véhicule le gaz-déchet au sein du système de purification de gaz entre un conduit de régénération et un conduit d'échappement.
   - le conduit de régénération est relié fluidiquement au second conduit d'interconnexion de manière à être alimenté en gaz-déchet.
   - le conduit d'échappement est relié fluidiquement à l'atmosphère afin de permettre d'y évacuer le flux gazeux de régénération, après sa traversé d'au moins un adsorbeur.
   - le circuit de régénération du système de purification de gaz comprend des passages ou conduits d'air et au moins un adsorbeur, de préférence les adsorbeurs.
   - le circuit de régénération comprend en outre au moins une vanne de contrôle, en particulier une ou des vannes rotatives.
   - le système de purification de gaz comprend en outre un circuit de recyclage de gaz.
   - le circuit de recyclage de gaz véhicule le flux de gaz expiré à purifier (i.e. mélange O₂/CO₂) et le flux de gaz purifié (i.e. O₂) au sein du système de purification de gaz entre un conduit d'entrée de gaz et un conduit de sortie.
   - le conduit d'entrée est relié fluidiquement à la ligne de récupération de gaz de manière à récupérer le flux de gaz expiré à purifier (i.e. mélange O₂/CO₂) amené par ladite ligne de récupération de gaz.
   - le conduit de sortie est raccordé fluidiquement à la ligne de recyclage de gaz pour l'alimenter en oxygène, i.e. en gaz recyclé, produit par l'unité de purification de gaz expiré.
   - le circuit de recyclage de gaz comprend des passages ou conduits d'air et au moins un adsorbeur, de préférence les adsorbeurs.
   - le circuit de recyclage comprend en outre au moins une vanne de contrôle, en particulier une ou des vannes rotatives.
   - ledit au moins un module de séparation électrochimique de l'unité de génération d'oxygène comprend une ou plusieurs membranes céramiques.
   - la ou les membranes céramiques sont dopées par un ou des électrolytes.
   - la première ligne de gaz de l'unité de génération d'oxygène comprend des moyens d'aspiration de gaz, tel un ventilateur, une soufflante, une pompe aspirante ou analogue.
   - les moyens de chauffage de gaz comprennent ou sont un dispositif de chauffage électrique.
   - les moyens de chauffage de gaz sont configurés pour préchauffer le flux d'air jusqu'à une température supérieure à 400°C, de préférence d'au moins 500°C, de préférence encore d'au moins 600°C.
   - les moyens de chauffage de gaz sont agencés entre les moyens d'aspiration de gaz et ledit au moins un module de séparation électrochimique.
   - des moyens d'échange thermique sont agencés sur la première ligne de gaz et sur la seconde ligne de gaz.
   - les moyens d'échange thermique comprennent un échangeur de chaleur (i.e. de calories), de préférence un échangeur de chaleur à contre-courant.
   - les moyens d'échange thermique sont agencés entre les moyens d'aspiration de gaz et les moyens de chauffage de gaz.
   - la seconde ligne de gaz de l'unité de génération d'oxygène est agencée en parallèle de la première ligne de gaz au sein desdits moyens d'échange thermique de manière à opérer un échange thermique entre le flux d'air circulant dans la première ligne de gaz et le gaz-déchet circulant dans la seconde ligne de gaz, de préférence un échange thermique à contre-courant.
   - les moyens d'échange thermique sont configurés pour assurer un transfert de calories du gaz-déchet véhiculé par la seconde ligne de gaz vers l'air véhiculé par la première ligne de gaz de manière à réchauffer l'air et à refroidir le gaz-déchet.
   - la seconde ligne de gaz de l'unité de génération d'oxygène convoie un gaz-déchet ayant une concentration en oxygène inférieure à 21% vol., de préférence inférieure ou égale à typiquement inférieure ou égale à 19% vol.
   - la seconde ligne de gaz de l'unité de génération d'oxygène convoie un gaz-déchet ayant une température d'au moins 250°C, typiquement d'au moins 300°C environ, de préférence après passage dans les moyens d'échange thermique (i.e. en sortie).
   - le système de purification de gaz comprend des seconds moyens de pilotage configurés pour piloter le fonctionnement du système de purification de gaz, en particulier les vannes de contrôle, i.e. les vannes rotatives.
   - un second conduit d'interconnexion relie fluidiquement la seconde ligne de gaz de l'unité de génération d'O₂ au système de purification de gaz.
   - le second conduit d'interconnexion véhicule le gaz-déchet entre l'unité de génération d'O₂ et le système de purification de gaz.
   - ledit au moins un module de séparation électrochimique de l'unité de génération d'oxygène comprend une ou plusieurs membranes céramiques.
   - les membranes céramiques sont dopées par un ou des électrolytes.
   - le ou les électrolytes comprennent ou sont par exemple des composés à base de Cérium.
- la (ou chaque) membrane céramique est configurée pour permettre aux molécules d'O₂ contenues dans le gaz, i.e. de l'air, de la traverser tout en retenant les molécules d'autres composés ou espèces gazeux, notamment celles d'azote, de CO₂, d'argon etc....
- l'unité de génération d'oxygène fournit de l'oxygène à une concentration d'au moins 99% en volume, de préférence d'au moins 99.5 % en volume, avantageusement entre 99.5% et 100%.
- le ou chaque module de séparation d'O₂ comprend une entrée d'air par lequel l'air véhiculé par la première ligne de gaz pénètre dans le ou chaque module de séparation d'O₂, avant sa séparation par la (ou les) membrane céramique agencée dans le ou chaque module de séparation d'O₂.
- le ou chaque module de séparation d'O₂ comprend une première sortie alimentant le premier conduit de sortie en un flux d'oxygène de haute pureté produit par la (ou les) membrane céramique.
- le ou chaque module de séparation d'O₂ comprend une seconde sortie alimentant la seconde ligne de gaz en gaz-déchet formé de composés gazeux n'ayant pas traversé, c'est-à-dire ayant été retenu, par la (ou les) membrane céramique, typiquement de l'azote, du CO₂, de l'argon etc....
- les premiers moyens de pilotage comprennent une première carte de commande électronique et une première unité de contrôle à microprocesseur(s), typiquement un (ou des) microcontrôleur.
- l'unité de génération d'O₂ comprend des premiers moyens de pilotage configurés pour piloter chaque module de séparation d'O₂.
- les premiers moyens de pilotage sont en outre configurés pour commander les moyens de chauffage et/ou les moyens d'aspiration des gaz.
- les premiers moyens de pilotage sont configurés pour alimenter électriquement la (ou les) membranes céramiques du ou de chaque module de séparation d'O₂
- les premiers moyens de pilotage sont alimentés électriquement par des moyens alimentation électrique, tel le secteur (110/220 V) et/ ou une batterie rechargeable ou analogue.
- l'interface respiratoire est un masque respiratoire ou analogue, en particulier un masque facial, i.e. bucco-nasal, couvrant le nez et la bouche de l'utilisateur.
- l'interface respiratoire comprend un orifice d'entrée d'oxygène et un orifice de sortie de gaz expiré, i.e. mélange O₂/CO₂.
- l'interface respiratoire est un masque respiratoire comprenant un orifice ou port d'entrée d'oxygène pour fournir le gaz riche en oxygène et un orifice ou port de sortie de gaz expiré pour évacuer le mélange gazeux CO₂/O₂ expiré par l'utilisateur.
- le masque respiratoire comprend un coussin souple venant en contact et assurant une étanchéité fluidique avec le visage de l'utilisateur.
- le masque respiratoire comprend un corps de masque, par exemple en polymère rigide, formant une coque délimitant une enceinte pour le gaz.
- le coussin souple est couplé mécaniquement au corps de masque, de préférence de manière détachable.
- le coussin souple comprend un passage central au sein duquel viennent se loger le nez et la bouche de l'utilisateur, lorsqu'il porte le masque facial.
- l'utilisateur respire le gaz contenu dans l'enceinte, c'est-à-dire inspire et expire le gaz dans l'enceinte du corps de masque.
- l'orifice d'entrée d'oxygène et l'orifice de sortie de gaz expiré sont aménagés dans le corps de masque.
- la ligne principale d'acheminement de gaz et/ou la ligne de récupération de gaz et/ou la ligne de recyclage de gaz sont ou comprennent une ou des conduites de gaz, de préférence souples, c'est-à-dire des tuyaux flexibles, par exemple en polymère.
- la ligne principale d'acheminement de gaz et/ou la ligne de récupération de gaz viennent se raccorder au corps de masque de manière à être en communication fluidique avec l'orifice d'entrée d'oxygène et l'orifice de sortie de gaz expiré.
- la ligne principale d'acheminement de gaz est raccordée fluidiquement à l'orifice d'entrée d'oxygène et la ligne de récupération de gaz est raccordée fluidiquement à l'orifice de sortie de gaz expiré de l'interface respiratoire.
- la ligne principale d'acheminement de gaz est raccordée fluidiquement à l'orifice d'entrée d'oxygène de l'interface respiratoire de manière à introduire du gaz respiratoire dans l'interface respiratoire.
- la ligne de récupération de gaz est raccordée fluidiquement à l'orifice de sortie de gaz expiré de l'interface respiratoire de manière à extraire un gaz expiré par le patient de l'interface respiratoire, typiquement un gaz contenant de l'oxygène et du CO₂, et habituellement de la vapeur d'eau.
- la ligne de recyclage de gaz est raccordée fluidiquement au réservoir-tampon pour l'alimenter en gaz purifié, c'est-à-dire en gaz recyclé.
- le gaz purifié riche en oxygène acheminé par la ligne de recyclage de gaz contient une proportion (i.e. teneur) d'oxygène inférieure ou égale au gaz provenant de la source de gaz, i.e. d'oxygène (quasi)pur, et acheminé par la ligne principale d'acheminement de gaz.
- le réservoir-tampon comprend un ballon flexible, un soufflet ou analogue.
- le réservoir-tampon comprend une valve d'échappement permettant d'évacuer à l'atmosphère tout excès de gaz, i.e. d'O₂, lorsque le réservoir-tampon est plein de gaz, i.e. d'oxygène.
- le réservoir-tampon est dimensionné pour contenir de 0.5 à 5 litres de gaz (à l'état non-comprimé).
- le réservoir-tampon est configuré pour permettre d'y réaliser un mélange de gaz à partir de gaz respiratoire riche en oxygène (de préf. >99% O₂) provenant de la ligne principale d'acheminement de gaz, tel de l'oxygène pur (i.e. env. 100%), et de gaz purifié, i.e. le gaz recyclé contenant au moins 99% d'oxygène, provenant de la ligne de recyclage de gaz.
- le réservoir-tampon est configuré pour alimenter la ligne principale d'acheminement de gaz avec de l'oxygène résultant du mélange de gaz opéré dans le réservoir-tampon.
- le système de purification de gaz comprend au moins un adsorbant présentant une sélectivité supérieure pour le CO₂ que pour l'O₂ de manière à adsorber au moins une partie du CO₂ contenu dans le mélange gazeux CO₂/O₂ expiré.
- le système de purification de gaz comprend au moins un adsorbant zéolitique , c'est-à-dire choisi parmi les zéolites échangées ou non échangées, et/ou au moins un adsorbant choisi parmi les silicates.
- le système de purification de gaz comprend plusieurs adsorbeurs contenant chacun au moins un adsorbant zéolitique ou silicate, ou leurs mélanges.
- le système de purification de gaz comprend au moins un adsorbant pour éliminer le CO₂ et la vapeur d'eau (H₂O), c'est-à-dire que le CO₂ et la vapeur d'eau sont éliminés sur un même lit d'adsorbant, par exemple un lit de zéolite unique, ou sur des lits successifs d'absorbants différents, par exemple un lit de silicate et un lit de zéolite.
- les adsorbeurs sont agencés en parallèle et fonctionnent de manière alternée.
- les adsorbeurs sont ou ont une forme de cartouche.
- le système de purification de gaz comprend trois adsorbeurs comprenant un premier adsorbeur ou cartouche, un second adsorbeur ou cartouche, et un troisième adsorbeur ou cartouche.
- chaque adsorbeur est configuré pour fonctionner selon des cycles d'adsorption/régénération de type PSA (Pressure Swing Adsorption), i.e. adsorption à pression modulée.
- pendant le fonctionnement du système de purification de gaz, l'un des adsorbeurs est en phase d'adsorption et l'un ou les deux autres adsorbeurs sont en phase de régénération.
- alternativement, pendant le fonctionnement du système de purification de gaz, un adsorbeur est en phase de d'adsorption, pendant que deux adsorbeurs sont en phase de régénération, c'est-à-dire qu'il y a toujours plus d'adsorbeurs en phase de régénération qu'en phase de d'adsorption.
- en phase d'adsorption (i.e. de production), l'adsorbant adsorbe, i.e. piège/retient, au moins une partie du CO₂ et de la vapeur d'eau (H₂O) contenus dans le mélange gazeux CO₂/O₂ expiré.
- en phase de régénération (i.e. phase de désorption), au moins une partie du CO₂ et éventuellement de la vapeur d'eau ayant été adsorbés par l'adsorbant est désorbée, i.e. libérée.
- en phase de régénération, la régénération de l'adsorbant par le flux de gaz-déchet provenant de l'unité de génération d'O₂ est opérée à contre-courant.
- le système de purification de gaz comprend en outre une ou plusieurs vannes de contrôle, aussi appelée vannes de distribution, agencées, sur le trajet du gaz, en amont et/ou en aval des adsorbeurs pour contrôler les entrées et les sorties de gaz desdits adsorbeurs, par exemple des vannes rotatives ou d'autres vannes.
- la ou les vannes de contrôle sont commandées par les seconds moyens de pilotage du système de purification de gaz.
- la ou les vannes de contrôle sont des vannes rotatives.
- l'installation comprend en outre des moyens alimentation électrique alimentant les composants de l'installation ayant besoin de courant électrique pour fonctionner, notamment les moyens de pilotage.
- le système de purification de gaz est agencé dans un boitier rigide.
- le circuit de recyclage comprend au moins un capteur de débit, en particulier capteur de débit massique
- au moins un capteur de débit est agencé dans le conduit d'entrée du circuit de recyclage.
- au moins un capteur de débit coopère avec les seconds moyens de pilotage du système de purification de gaz, en particulier en lui fournissant des mesures de débit.

Selon un autre aspect, l'invention porte aussi sur un procédé de régénération d'un ou plusieurs adsorbeurs du système de purification de gaz d'une installation de fourniture de gaz respiratoire selon l'invention, en particulier l'installation de fourniture de gaz respiratoire décrite ci-dessus, dans lequel :
a) récupère au moins une partie du gaz-déchet généré par au moins un module de séparation électrochimique de l'unité de génération d'O₂ ;
b) on convoie le gaz-déchet jusqu'au système de purification de gaz ; et
c) on met en contact le gaz-déchet avec au moins un adsorbant contenu dans au moins un adsorbeur du système de purification pour régénérer le dit au moins un adsorbeur.

Selon le mode de réalisation considéré, le procédé de régénération de l'invention peut comprendre l'une ou plusieurs des caractéristiques suivantes :
- le gaz-déchet comprend moins de 21% vol. d'oxygène.
- à l'étape c), le gaz-déchet est à une température d'au moins 250°C, de préférence d'au moins 300°C,
- le gaz-déchet est refroidi par échange thermique avec un flux d'air avant l'étape c).
- le gaz-déchet est obtenu par séparation d'air dans ledit au moins un module de séparation électrochimique avec production, en outre, d'un flux d'oxygène.
- ledit au moins un adsorbant est régénéré à contre-courant.
- le flux de gaz-déchet traverse ledit au moins un adsorbant pour le régénérer.
- après son passage au travers dudit au moins un adsorbant, le flux de gaz-déchet est rejeté vers l'atmosphère. Il comprend les composés désorbés de l'adsorbant, notamment du CO₂ et de la vapeur d'eau.

L'invention va maintenant être mieux comprise grâce à la description détaillée suivante, faite à titre illustratif mais non limitatif, en référence aux figures annexées parmi lesquelles :
Fig. 1 schématise un mode de réalisation d'une installation de fourniture d'O₂ selon la présente invention.
Fig. 2 schématise un mode de réalisation de l'unité de génération d'O₂ de l'installation de Fig. 1.
Fig. 3 schématise un mode de réalisation de l'architecture de l'unité de purification des gaz expirés de l'installation de Fig. 1.
Fig. 4 représente les sous-ensembles d'une vanne de rotation servant à distribuer les gaz dans l'unité de purification des gaz expirés de Fig. 3.
Fig. 5 représente la vanne de rotation servant à distribuer les gaz dans l'unité de purification des gaz expirés de Fig. 3.

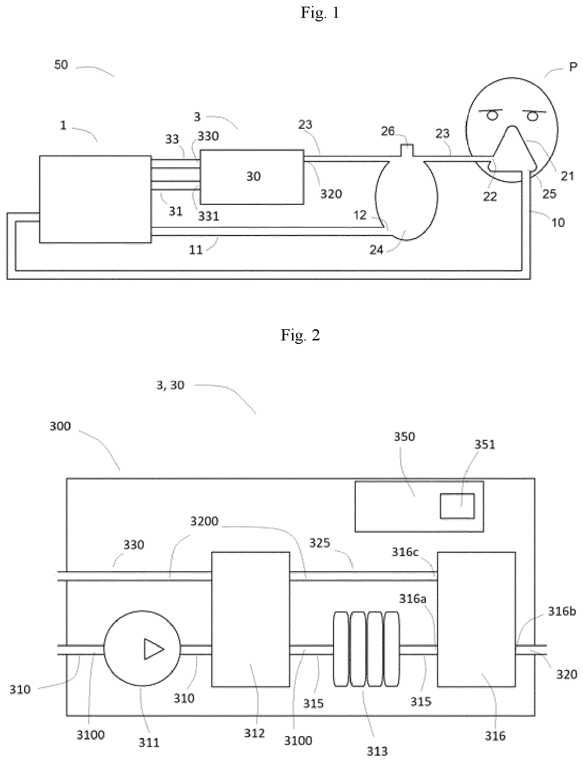

Fig. 1 schématise un mode de réalisation d'une installation 50 de fourniture d'un mélange gazeux riche en O₂ selon la présente invention comprenant une unité de génération d'oxygène 30 en tant que source de gaz 3 fournissant un gaz respiratoire riche en oxygène (teneur en O₂ >99% en vol) à une ligne principale d'acheminement de gaz 23, aussi appelée circuit patient, pour acheminer le flux de gaz respiratoire de la source de gaz 3 à une interface respiratoire 21, tel un masque ou analogue, permettant d'administrer le gaz à un utilisateur P, i.e. un patient, lors de ses phases inspiratoires, c'est-à-dire lorsqu'il inhale du gaz.

Le fonctionnement et l'architecture de l'unité de génération d'oxygène 30 sont détaillés ci-après.

L'installation 50 comprend aussi une ligne de récupération de gaz expiré 10 reliée fluidiquement à l'interface respiratoire 21 qui permet de récupérer et d'acheminer tout ou partie du mélange gazeux CO₂/O₂ expiré par le patient P, lors de ses phases expiratoires, lequel se retrouve dans l'interface respiratoire 21, lorsque le patient P exhale.

La ligne de récupération de gaz 10 alimente en mélange gazeux CO₂/O₂ expiré, un système de purification de gaz 1 servant à éliminer la majorité et, de préférence (quasi-) tout, le CO₂ contenu dans le mélange gazeux CO₂/O₂ expiré et obtenir ainsi un gaz purifié contenant essentiellement de l'oxygène, i.e. O₂.

Une ligne de recyclage de gaz 11 reliée fluidiquement au système de purification de gaz 1 permet de récupérer l'oxygène fourni par le système de purification de gaz 1 et l'amène ensuite jusqu'à la ligne principale d'acheminement de gaz 23, via un réservoir-tampon 24. Le réservoir-tampon 24 est donc agencé sur la ligne principale d'acheminement de gaz 23, notamment alimenté par celle-ci, et par ailleurs alimenté par la ligne de recyclage de gaz 11.

La ligne principale d'acheminement de gaz 23, aussi appelé circuit patient, comprend un tuyau flexible ou analogue, est raccordée fluidiquement, via son extrémité aval, à l'orifice ou port d'entrée 22 de l'interface respiratoire 21, tel un masque facial, de manière à alimenter le patient P en O₂ provenant du réservoir-tampon 24.

Le réservoir-tampon 24 sert de réserve de gaz respiratoire, i.e. d'oxygène. Il est en communication fluidique non seulement avec l'unité de génération d'O₂ 30 mais aussi avec le système de purification de gaz 1. Dit autrement, le réservoir-tampon 24 est alimenté par un premier flux d'oxygène (quasi-)pur (teneur égale à env. 100% O₂) convoyé par la ligne principale d'acheminement de gaz 23 provenant de l'unité de génération d'O₂ 30 et par un second flux d'oxygène purifié recyclé provenant de la ligne de recyclage 11 alimentée par le système de purification de gaz 1, de préférence de l'oxygène purifié ayant une pureté de l'ordre de 99% ou plus, comme expliqué ci-après. Les deux flux d'oxygène se mélangent donc dans le réservoir-tampon 24.

Lorsque le patient P respire, le réservoir-tampon 24 qui est rempli d'oxygène, satisfait la demande instantanée du patient P en lui fournissant au moins une partie de l'oxygène qu'il contient, i.e. de l'oxygène (quasi-)pur (avec impuretés inévitables possibles).

Par ailleurs, le gaz expiré, i.e. un mélange gazeux CO₂/O₂, par le patient P ressort de l'interface respiratoire 21 par un port ou orifice de sortie 25 aménagé dans l'interface respiratoire 21, avant d'être récupéré et convoyé par la ligne de récupération de gaz 10 formant un circuit expiratoire, tel un conduit ou tuyau de gaz flexible, connecté fluidiquement au port de sortie 25 de l'interface respiratoire 21.

La ligne de récupération de gaz 10 permet donc de récupérer et acheminer (au moins une partie) le mélange gazeux CO₂/O₂ expiré par l'utilisateur se retrouvant dans l'interface respiratoire 21, lors des phases expiratoires de l'utilisateur P, jusqu'au système de purification de gaz 1 où il est purifié, comme expliqué ci-avant.

Dans tous les cas, le mélange gazeux CO₂/O₂ expiré par le patient P contient encore une forte proportion d'O₂ non-métabolisé, typiquement au moins 90 à 95% d'O₂ résiduel, alors que sa teneur en dioxyde de carbone CO₂ issu de la respiration cellulaire et des échanges pulmonaires est faible, typiquement de l'ordre de 5 % vol. Le gaz expiré peut aussi contenir d'autres composés gazeux ou impuretés inévitables, en particulier de la vapeur d'eau (H₂O), voire de l'azote et/ou de l'argon en quantité négligeable pouvant résulter par exemple de défauts d'étanchéité au niveau de l'interface respiratoire 21, notamment sur les contours du masque.

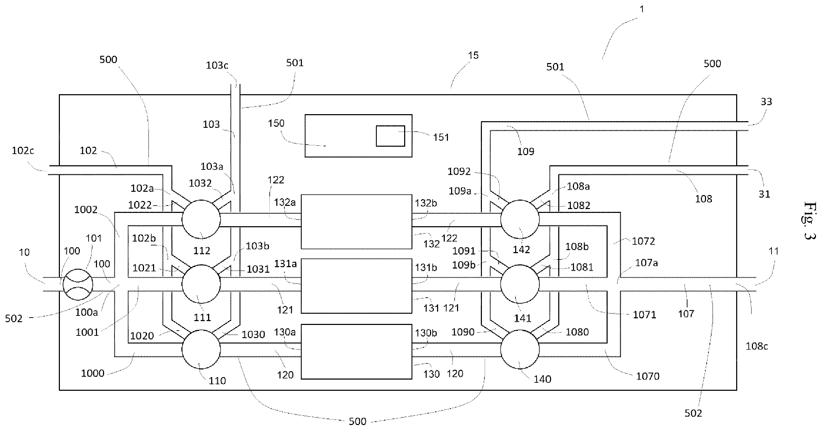

Comme déjà dit, le flux gazeux de mélange CO₂/O₂ est acheminé par la ligne de récupération de gaz 10 jusqu'à l'unité de purification 1 des gaz expirés au sein de laquelle le CO₂ et préférentiellement la vapeur d'eau sont épurés/éliminés, notamment adsorbés, i.e. captés, par un (ou des) lit d'adsorbants 130-132 de l'unité de purification 1, comme expliqué ci-après et illustré en Fig. 3.

Il est à noter que, dans le cadre de la présente invention, on utilise indifféremment la terminologie « le gaz expiré » ou « les gaz expirés » (i.e. singulier ou pluriel) pour désigner le mélange gazeux rejeté par les poumons du patient, c'est-à-dire expiré, lequel contient majoritairement de l'O₂ (e.g. >90% vol.), du CO₂ (e.g. de l'ordre de 5% vol. environ) et généralement de la vapeur d'eau, voire d'autres impuretés éventuelles inévitables.

Le gaz purifié, i.e. de l'oxygène (quasi) pur produit par l'unité de purification 1 contient essentiellement de l'oxygène et des quantités faibles, voire négligeables, de CO₂ et éventuellement de vapeur d'eau (i.e. humidité) qui n'ont pas été éliminés. Dans tous les cas, le gaz purifié, i.e. de l'oxygène, a, après purification, une teneur en oxygène très supérieure à celle du gaz expiré (i.e. mélange O₂/CO₂), typiquement l'oxygène purifié a une pureté d'au moins 99% vol. environ.

En effet, le système de purification de gaz 1, qui est alimenté en mélange gazeux CO₂/O₂ expiré (i.e. teneur O₂ < 95% env.) par la ligne de récupération de gaz 10, permet d'éliminer la majeure partie du CO₂ et préférentiellement de la vapeur d'eau contenue dans le mélange gazeux CO₂/O₂ expiré et obtenir un gaz purifié formé essentiellement d'oxygène (quasi-)pur (e.g. teneur O₂ > 99.90% env., voire >99.95% vol.).

En aval du système de purification de gaz 1, une ligne de recyclage de gaz 11 reliée fluidiquement au système de purification de gaz 1 et par ailleurs à la ligne principale d'acheminement de gaz 23, via le réservoir-tampon 24, permet de recueillir et acheminer le gaz purifié contenant essentiellement de l'oxygène (i.e. teneur O₂ > 99.9% env.) produit par le système de purification de gaz 1 et le réinjecter dans la ligne principale d'acheminement de gaz 23, c'est-à-dire dans le flux d'oxygène provenant de l'unité de génération d'O₂ 3. L'oxygène provenant du système de purification de gaz 1 se mélange donc avec le flux d'oxygène provenant de l'unité de génération d'oxygène 30, comme expliqué ci-dessous.

La quantité de composés CO₂ et vapeur d'eau résiduels dans le gaz purifié provenant du système de purification de gaz 1 sont négligeables, typiquement <0.1% vol., c'est-à-dire que le gaz purifié est essentiellement constitué d'O₂ quasi-pur.

Le gaz purifié, i.e. oxygène, est donc acheminé par la ligne de recyclage de gaz 11 jusqu'au réservoir 24 et y pénètre via un port d'entrée 12 auquel est raccordée fluidiquement la ligne de recyclage de gaz 11.

Le système de purification de gaz 1 constitue donc une unité d'épuration de gaz expirés permettant de purifier et recycler ensuite les gaz expirés riche en O₂ en les débarrassant du CO₂ et éventuellement de la vapeur d'eau (H₂O) qu'ils contiennent, au lieu de les rejeter à l'atmosphère, comme c'est habituellement le cas, et ainsi gaspiller l'oxygène qui s'y trouve encore en grande quantité, typiquement au moins 90% vol. environ.

Comme illustré en Fig. 2, l'unité de génération d'oxygène 30 comprend un ou des modules de séparation électrochimique 316 permettant de fournir le gaz respiratoire riche en oxygène, typiquement de l'oxygène pur ou quasiment pur, par exemple ayant une pureté >99.95%vol., à la ligne principale d'acheminement de gaz 23, c'est-à-dire le circuit patient, puis au réservoir-tampon 24 alimentant l'interface respiratoire 21, tel un masque facial ou analogue, de manière à administrer le gaz respiratoire, i.e. oxygène, au patient P, lors de ses phases inspiratoires.

Un exemple d'un module de séparation électrochimique 316 utilisable dans le cadre de la présente invention est décrit par le document : 49th International Conference on Environmental Systems; 7-11 July 2019; Boston (MA); ICES-2019-379; Solid State Electrochemical Oxygen Separation and Compression; M. Reisert et al.; p. 1-10*.*

Fig. 2 schématise l'architecture et le fonctionnement d'un mode de réalisation de l'unité de génération d'O₂ 3 intégrant un module de séparation électrochimique 316.

Cette unité de génération d'O₂ 3 comprend des premiers moyens de pilotage 350, 351 comprenant par exemple une première carte de commande électronique 350 et une première unité de contrôle 351 à microprocesseur(s), typiquement un (ou des) microcontrôleur. Les éléments électromécaniques de l'unité de génération d'O₂ 3 sont alimentés électriquement et commandés par les premiers moyens de pilotage 350, 351.

L'unité de génération d'O₂ 3 comprend en outre une première ligne de gaz interne 3100 comprenant une portion amont appelée conduit d'admission 310 et une portion aval appelée conduit d'entrée 315; et une seconde ligne de gaz interne 3200 comprenant une portion amont appelée second conduit de sortie 325 et une portion aval appelée conduit d'échappement 330.

Des moyens d'échange thermique 312, tel un échangeur de chaleur, 312 sont agencés sur la première ligne de gaz interne 3100 de sorte que le flux gazeux véhiculé par la première ligne de gaz interne 3100 puisse traverser ces moyens d'échange thermique 312, avant d'atteindre des moyens de chauffage de gaz 313 agencés sur le conduit d'entrée 315, c'est-à-dire en aval des moyens d'échange thermique 312 en considérant le sens de circulation du flux gazeux.

La première ligne de gaz interne 3100 vient se raccorder au système de purification de gaz, via un premier conduit d'interconnexion 31, e.g. tuyau, conduit, ligne ou analogue, alors que la seconde ligne de gaz interne 3200 vient se raccorder au système de purification de gaz, via un second conduit d'interconnexion 33, e.g. tuyau, conduit, ligne ou analogue, comme détaillé ci-après.

Le conduit d'admission 310 interne de la première ligne de gaz interne 3100 de l'unité de génération d'O₂ 3 comprend des moyens d'aspiration de gaz 311, tel qu'un dispositif ventilateur de type soufflante ou analogue, permettant d'aspirer le gaz fourni par le premier conduit d'interconnexion 31, comme expliqué ci-après, pour le faire circuler dans le conduit d'admission 310 interne et ensuite au travers des moyens d'échange thermique 312, dans lesquels un échange thermique entre le flux de gaz et le flux de gaz-déchet peut avoir lieu, comme expliqué ci-après.

Après passage dans les moyens d'échange thermique 312, le flux de gaz est récupéré et convoyé par le conduit d'entrée 315 de la première ligne de gaz interne 3100, jusqu'à des moyens de chauffage de gaz 313, tel qu'un réchauffeur de gaz ou un dispositif de chauffage analogue.

Les moyens d'échange thermique 312 sont donc agencés, sur la première ligne de gaz interne 3100, entre les moyens d'aspiration de gaz 311 et les moyens de chauffage de gaz 313.

Comme détaillé ci-après, la première ligne de gaz interne 3100 permet d'amener le flux d'air fourni par le système de purification de gaz 1, via un premier conduit d'interconnexion ligne 31, jusqu'au(x) module(s) de séparation d'O₂ électrochimique 316 de l'unité de génération d'O₂ 3, lesquels sont agencés en aval des moyens de chauffage de gaz 313 sur la première ligne de gaz interne 3100.

Les moyens de chauffage de gaz 313, par exemple un dispositif de chauffage électrique de gaz, sont configurés pour préchauffer le flux de gaz, à savoir de l'air, amené par le conduit d'entrée 315 de la première ligne de gaz interne 3100 jusqu'à une température supérieure à 600°C avant qu'il ne soit fourni au(x) module(s) de séparation d'O₂ électrochimique 316. Selon un autre mode de réalisation, on prévoit plusieurs modules de séparation d'O₂ électrochimique 316, par exemple agencés en parallèle.

Le ou chaque module de séparation d'O2 316 comprend une ou plusieurs membranes céramiques dopées par un (ou des) électrolyte(s) qui, sous l'action de la chaleur et d'un potentiel électrique appliqué par la première carte de commande 350, permet aux molécules d'O₂ contenues dans le flux d'air provenant du système de purification de gaz 1, de traverser la ou les membranes céramiques et d'être récupérées dans le premier conduit de sortie 320 qui alimente alors la ligne principale d'acheminement de gaz 23 de l'installation 50 de l'invention, en un flux d'oxygène de haute pureté, i.e. > 99,50%vol., en général d'une pureté de l'ordre de 99,90%vol à 99.95%vol.

L'air alimentant le (ou les) module de séparation d'O2 316 est capté à l'atmosphère via un conduit d'admission d'air 102 du système de purification de gaz 1, qui est relié à l'atmosphère ambiante, puis véhiculé au travers du système de purification de gaz 1, au travers de l'un ou l'autre des adsorbeurs 130-132, comme expliqué ci-dessous et visible en Fig. 3.

Autrement dit, le ou chaque module de séparation d'O2 316 comprend :
- une entrée de gaz 316a par lequel le gaz véhiculé, i.e. le flux d'air, par la première ligne de gaz interne 3100 pénètre dans le ou chaque module de séparation d'O2 316, avant sa séparation par la (ou les) membrane céramique qui s'y trouve ;
- une première sortie 316b alimentant le premier conduit de sortie 320 en un flux d'oxygène de haute pureté produit par la (ou les) membrane céramique ; et
- une seconde sortie 316c alimentant la seconde ligne de gaz interne 3200 en gaz-déchet n'ayant pas traversé, c'est-à-dire ayant été retenu, par la (ou les) membrane céramique.

Le flux d'oxygène convoyé par le conduit de sortie 320 est formée d'oxygène de (très) haute pureté, par exemple d'au moins 99.95% vol. à 99.99% vol. environ, à température ambiante, i.e. de l'ordre de 10 à 30°C, c'est-à-dire de l'oxygène ne contenant pas ou des quantités négligeables d'autres composés en tant qu'impuretés inévitables éventuelles, tel de l'azote (N₂) et/ou de l'argon (Ar). Autrement dit, la ou les membranes céramiques sont conçues pour ne laisser passer que les molécules d'oxygène qui sont ensuite récupérées et fournies par le conduit de sortie 320 à la ligne principale d'acheminement de gaz 23 qui achemine ce flux d'oxygène jusqu'au réservoir-tampon 24, comme déjà expliqué.

Le réservoir-tampon 24 est préférentiellement muni d'une valve d'échappement 26 permettant d'évacuer à l'atmosphère ambiante tout excès d'O₂ lorsque le réservoir-tampon 24 est plein d'oxygène, c'est-à-dire totalement rempli.

Dans tous les cas, récupérer la majeure partie de l'O₂ formant le composé fortement majoritaire dans les gaz expirés par le patient P (i.e. mélange CO₂/O₂), et la recycler après purification, permet d'éviter son gaspillage, et donc ainsi de limiter le débit d'oxygène fourni par la source d'O₂ 3.

Par exemple, on considère que la consommation métabolique d'O₂ d'un être humain est de l'ordre de 0.5 L/min. Autrement dit, si un patient a une ventilation minute de 10 L/min, i.e. inhale 10L de gaz en 1 minute, d'O₂ pur, c'est-à-dire à teneur de 100% d'O₂, alors 9.5 L d'O₂ sont expirés, le reste (0.5 L) est constitué de CO₂ et de vapeur d'eau. Dès lors, le système de purification de gaz 1 permet de récupérer environ 9.5L d'O₂. Par ailleurs, grâce à l'unité de génération d'O₂ 3 fournissant un débit d'O₂ de 1 L/min et au recyclage de l'O₂ contenu dans le gaz expiré et son mélange avec l'O₂ provenant de l'unité de génération d'O₂ 3, le débit d'O₂ obtenu excède la ventilation minute du patient.

Par ailleurs, la quasi-totalité des autres composés présents dans l'air amené par la première ligne de gaz interne 3100, tel l'azote, l'argon et/ou autres composés éventuels, ressortent du module de séparation d'O₂ 316 par la seconde sortie 316c sous forme d'un gaz-déchet et sont ensuite convoyés par la seconde ligne de gaz interne 3200 pour servir de gaz de régénération des adsorbeurs du système de purification de gaz 1, comme détaillé ci-après.

Plus précisément, du fait de la combinaison du gaz préchauffé et de l'effet Joule se manifestant dans le ou chaque module de séparation d'O₂ 316, le gaz-déchet contenant les autres composés gazeux (N₂, Ar...), qui est à une température de l'ordre de 850°C, ressort du module de séparation d'O₂ 316 par le second conduit de sortie 325 formant la portion amont de la seconde ligne de gaz interne 3200, lequel convoie le gaz-déchet au travers des moyens d'échange thermique 312, tel un échangeur de chaleur, qui est aussi agencé sur la seconde ligne de gaz interne 3200.

Dit autrement, les moyens d'échange thermique 312 sont traversés par la première ligne de gaz interne 3100 et la seconde ligne de gaz interne 3200, comme visible en Fig. 2 de manière à pouvoir opérer entre les flux convoyés par ces lignes, un échange thermique de préférence à contre-courant. Le flux de gaz-déchet en ressort alors par le conduit d'échappement 330 formant la portion aval de la seconde ligne de gaz interne 3200, et est ensuite convoyé jusqu'au système de purification de gaz 1, comme détaillé ci-après.

Les moyens d'échange thermique 312 sont configurés pour récupérer une partie de l'énergie calorifique, c'est-à-dire des calories, du gaz-déchet chaud qui est typiquement à une température très élevée supérieure à 500°C, par exemple à 850°C environ, amené par le second conduit de sortie 325 de la seconde ligne de gaz interne 3200 et de la fournir au gaz, c'est-à-dire l'air, circulant dans le conduit d'admission 310 de la première ligne de gaz interne 3100 pour le préchauffer et optimiser ainsi la consommation électrique de l'unité de génération d'O₂ 3. Autrement dit, il s'opère un échange thermique au sein des moyens d'échange thermique 312, de préférence à contre-courant, entre le flux de gaz circulant dans le conduit d'admission 310 et le flux de gaz-déchet amené par le second conduit de sortie 325.

Après échange thermique, i.e. de l'échangeur de chaleur/calories, le gaz-déchet évacué par le conduit d'échappement 330 est à une température plus basse mais encore élevée, typiquement de l'ordre de 250 à 300°C environ, et contient moins de 21%vol. d'O₂ puisque de l'oxygène présent dans le flux initial a migré à travers le module de séparation d'O₂ 316 et a été évacué successivement par le premier conduit de sortie 320 et la ligne principale d'acheminement de gaz 23 vers le réservoir-tampon 24. En effet, comme déjà indiqué, le premier conduit de sortie 320 de l'unité de génération d'O₂ 3 est connecté fluidiquement à la ligne principale d'acheminement de gaz 23, incluant le réservoir-tampon 24, comme illustré en Fig. 1.

Le débit d'O₂ fourni par l'unité de génération d'O₂ 3 est habituellement faible, par exemple de l'ordre de 1 L/min, de manière à limiter le poids et l'encombrement de l'unité de génération d'O₂ 3 mais aussi sa consommation électrique.

Tous les composants de l'unité de génération d'O₂ 3 sont avantageusement agencés dans une carcasse ou un boitier 300 de protection rigide.

Etant donné que le système de purification de gaz 1 est fluidiquement connecté à l'unité de génération d'O₂ 3 au moyen de deux conduits parallèles, à savoir un premier conduit d'interconnexion 31 et un second conduit d'interconnexion 33 reliés fluidiquement, respectivement, au conduit d'admission 310 et au conduit d'échappement 330, il est non seulement possible d'alimenter l'unité de génération d'O₂ 3 avec de l'air ayant traversé le système de purification de gaz 1, notamment l'un ou l'autre de ses adsorbeurs 130-132 afin de les refroidir comme expliqué ci-après, mais aussi de pouvoir utiliser le gaz-déchet généré par l'unité de génération d'O₂ 3 en tant que fluide de régénération du ou desdits adsorbeurs 130-132 afin de les régénérer comme détaillé ci-après.

Fig. 3 schématise un mode de réalisation du système de purification de gaz 1, aussi appelé « unité d'épuration de gaz expiré », faisant partie de l'installation 50 de fourniture de gaz respiratoire, i.e. de l'oxygène, selon l'invention, telle qu'illustrée en Fig. 1.

Outre les éléments déjà mentionné, le système de purification de gaz 1 ou unité d'épuration de gaz expiré comprend des seconds moyens de pilotage 150, 151, typiquement une seconde carte de commande électronique 150 et une seconde unité de contrôle 151 à microprocesseur(s), typiquement un (des) microcontrôleur.

Tous les éléments électromécaniques du système de purification 1 sont alimentés électriquement et commandés par les seconds moyens de pilotage 150, 151. Les seconds moyens de pilotage 150, 151 sont eux-mêmes alimentés électriquement par une source de courant électrique (non montrée), par exemple une liaison au courant du secteur (110/220V) de type cordon électrique et prise de raccordement, ou une (ou des) batterie d'alimentation électrique, de préférence rechargeable, et/ou un transformateur de courant.

Les seconds moyens de pilotage 150, 151 contrôlent notamment les vannes de contrôle, savoir les vannes rotatives 110-112, 140-142.

Plus précisément, la seconde carte de commande 150, i.e. une carte électronique, intègre préférentiellement l'unité de contrôle 151 à microprocesseur et est configurée pour commander et par ailleurs analyser les signaux provenant des différents composants de l'unité d'épuration de l'air expiré 1, tels que vannes, pompe, capteurs...

La seconde carte de commande 150 et les autres composants de l'unité de purification 1 sont, eux aussi, agencés dans une carcasse ou boitier 15 externe rigide, par exemple en polymère.

L'unité d'épuration de gaz expiré 1 comprend un conduit ou passage d'entrée 100 avec orifice d'entrée qui est relié fluidiquement à la ligne de récupération de gaz 10, aussi appelée circuit expiratoire, tel un conduit, un tuyau flexible ou analogue, servant à recueillir et à convoyer le gaz expiré par le patient (i.e. mélange O₂/CO₂) en sortie d'interface respiratoire 21, comme expliqué ci-avant.

La ligne de récupération de gaz 10 vient se raccorder fluidiquement, par son extrémité aval, au conduit d'entrée 100 via un système de raccordement comprenant par exemple des connecteurs réciproques de type mâle/femelle, permettant d'assurer une connexion mécanique et fluidique et par ailleurs à l'interface respiratoire 21, tel un masque facial par exemple, par son extrémité amont, afin d'y prélever le gaz expiré par le patient P.

Le gaz expiré (i.e. mélange CO/O₂) par le patient qui contient essentiellement de l'O₂ (de l'ordre de 90 à 95% vol. env.) et du CO₂ (de l'ordre de 5% vol. env.), voire de la vapeur d'eau et/ou d'autres impuretés inévitables, est purifié dans le système de purification de gaz 1 par élimination du CO₂ et préférentiellement de la vapeur d'eau présente, de manière à obtenir un gaz purifié contenant une forte proportion d'oxygène, par exemple au moins 99% à 99.9% vol. d'oxygène.

Autrement dit, l'entrée du gaz expiré par le patient dans l'unité d'épuration de gaz expiré 1, se fait via la portion amont ou portion d'entrée du conduit d'entrée 100 de l'unité d'épuration de gaz expiré 1.

Le conduit d'entrée 100 comprend par ailleurs un capteur de débit 101, typiquement un capteur de débit massique et, en aval de ce capteur de débit 101, le conduit d'entrée 100 se ramifie en un site d'embranchement 100a en trois sous-conduits, de préférence identiques, respectivement appelés sous-conduits d'entrée 1000, 1001, 1002. Le premier sous-conduit d'entrée 1000 débouche sur une première vanne rotative 110, le second sous-conduit d'entrée 1001 débouche sur une seconde vanne rotative 111 et le troisième sous-conduit d'entrée 1002 débouche sur une troisième vanne rotative 112. Les vannes rotatives 110, 111 et 112 sont identiques et leur fonctionnement sera explicité ci-après.

Par ailleurs, l'unité d'épuration de gaz expiré 1 comprend un conduit d'admission d'air 102 relié à l'atmosphère ambiante, via un port d'entrée d'air 102c et se sous-divisant (en 102a, 102b) en un premier sous-conduit d'admission 1020, un second sous-conduit d'admission d'air 1021 et un troisième sous-conduit d'admission 1022.

Le conduit d'admission d'air 102 permet de prélever l'air qui est, après avoir traversé le ou les adsorbeurs 130-132 de l'unité d'épuration de gaz expiré 1, comme expliqué ci-après, envoyé vers l'unité de génération d'O₂ 3 via le conduit d'interconnexion 31 alimentant fluidiquement le conduit d'admission 310 de l'unité de génération d'O₂ 3 avec l'air utilisé dans le module de séparation d'O₂ 316 pour produire de l'oxygène (quasi)pur.
Les premier, deuxième et troisième sous-conduits d'admission 1020, 1021, 1022 débouchent respectivement sur les première, deuxième et troisième vannes rotatives 110, 111, 112.

De façon similaire, l'unité d'épuration de gaz expiré 1 comprend un conduit d'échappement 103 relié à l'air ambiant en un port d'évacuation 103c, lequel est formé par réunion (en 103a et 103b) de trois sous-conduits 1030-1032, à savoir respectivement un premier, un second et un troisième sous-conduit d'échappement 1030, 1031, 1032. Comme précédemment, les premier, deuxième et troisième sous-conduits d'échappement 1030, 1031, 1032 débouchent respectivement sur les première, deuxième et troisième vannes rotatives 110, 111, 112. Le conduit d'échappement 103 ne possède aucune communication fluidique autre que celles le reliant à ses sous-conduits.

Chacun des sous-conduits issus des conduits d'entrée 100, d'admission 102 et d'échappement 103 débouchent sur l'une des première, seconde ou troisième vanne rotative 110, 111 ou 112. Par exemple, le premier sous-conduit d'entrée 1000 du conduit d'entrée 100, le premier sous-conduit d'admission 1020 du conduit d'admission 102 et le premier sous-conduit d'échappement 1030 du conduit d'échappement 103 débouchent sur la première vanne rotative 110. Il en va de manière analogue pour les autres sous-conduits 1001, 1021, 1031 ; 1002, 1022, 1032 et autres vannes 111 ; 112, respectivement.

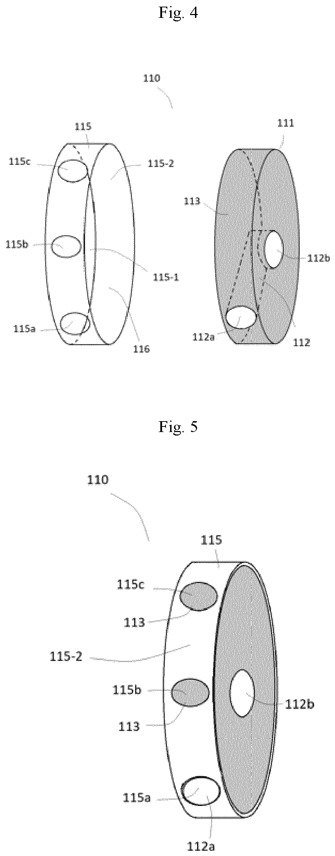

Fig. 4 est une vue schématique éclatée de la première vanne rotative 110. Elle comprend deux éléments principaux assemblés l'un à l'autre, à savoir un élément central 111 formant un cylindre plein traversé par un conduit coudé 112 reliant un orifice latéral 112a à un orifice central 112b ; et un élément périphérique 115 formant un « couvercle ». Il comprend une paroi de fond 115-1 en forme de disque et une paroi périphérique 115-2 formant une bordure annulaire périphérique autour de la paroi de fond. La paroi de fond 115-1 et la paroi périphérique 115-2 délimitent un espace ou logement central 116 configuré et dimensionné pour loger l'élément central 111, i.e. cylindre plein, après assemblage. L'élément périphérique 115 présente trois évidements latéraux 115a, 115b et 115c aménagé dans la bordure annulaire et en communication fluidique avec le logement central 116.

Les deuxième et troisième vanne rotative 111, 112 ont des structures identiques et ne sont donc pas détaillées.

Fig. 5 illustre l'assemblage de l'élément central 111 et élément périphérique 115 formant un « couvercle » de manière à obtenir ainsi la première vanne rotative 110. Dans cette configuration, l'orifice latéral 112a du conduit 112 du cylindre plein formant l'élément central 111 est en vis-à-vis de l'orifice latéral 115a de l'élément périphérique 115 formant couvercle. A contrario, les orifices latéraux 115b, 115c de l'élément périphérique 115 sont occultés par la paroi latérale 113, i.e. la paroi périphérique 115-2, de l'élément central, i.e. cylindre plein. L'orifice latéral 115a est donc en communication fluidique avec l'orifice central 112b du conduit 112, tandis que les orifices latéraux 115b, 115c de l'élément périphérique 115 sont « bouchés » par la paroi latérale 113 de l'élément central 111.

L'absence de fuites entre l'élément périphérique 115, l'élément central 111 et leurs orifices respectifs, peut être assurée en intégrant des joints (non représentés), par exemple des joints toriques, autour des orifices latéraux 115a, 115b, 115c de de l'élément périphérique 115.

En outre, les orifices 115a, 115b, 115c de ladite première vanne rotative 110 sont respectivement raccordés, par exemple emmanchés à force, soudés ou autre, au premier sous-conduit d'entrée 1000 du conduit d'entrée 100, au premier sous-conduit d'admission 1020 du conduit d'admission 102 et au premier sous-conduit d'échappement 1030 du conduit d'échappement 103.

Par ailleurs, l'orifice central 112b du conduit coudé 112 de l'élément central 111 est lui-même raccordé à un premier conduit 120 comme illustré en Fig. 3. Cette liaison est de type circulaire en ce sens que l'élément central 111 ayant une forme de cylindre plein, peut effectuer un mouvement de rotation autour de l'axe défini par l'orifice central 112b, physiquement raccordé au premier conduit 120. Un tel mouvement de rotation peut être obtenu par le couplage mécanique à un actionneur rotatif (non montré), par exemple un moteur pas-à-pas contrôlé par la seconde carte de commande 150, permettant alors à l'élément central 111 d'effectuer un mouvement de rotation dans l'élément périphérique 115.

Moyennant un contrôle approprié par la seconde carte de commande 150, on peut configurer la première vanne rotative 110 de manière à sélectionner une configuration fluidique, c'est-à-dire, sur la Fig. 5, de connecter fluidiquement le premier sous-conduit d'entrée 1000 du conduit d'entrée 100 au premier conduit 120, alors que le premier sous-conduit d'admission 1020 du conduit d'admission 102 et le premier sous-conduit d'échappement 1030 du conduit d'échappement 103 sont bouchés et ne peuvent pas véhiculer de gaz. En d'autres termes, la première vanne rotative 110 est configurée par exemple pour réaliser une communication fluidique entre le conduit d'entrée 100 et le premier conduit 120, et donc l'intégralité du gaz circulant dans le conduit d'entrée 100 est acheminé dans le premier conduit 120.

Une telle configuration peut être modifiée par la seconde carte de commande 150 de manière à connecter fluidiquement le premier sous-conduit d'admission 1020 du conduit d'admission 102 au premier conduit 120, ou le premier sous-conduit d'échappement 1030 du conduit d'échappement 103 au même premier conduit 120.

En d'autres termes, en fonction de la configuration de la première vanne rotative 110, le premier conduit 120 est fluidiquement connecté, soit au conduit d'entrée 100, soit au conduit d'admission 102, soit au conduit d'échappement 103.

Comme indiqué précédemment, les première, seconde et troisième vannes rotatives 110, 111, 112 sont identiques. Il apparait ainsi que la seconde vanne rotative 111 peut, en fonction de la configuration déterminée par la seconde carte de commande 150, mettre en relation fluidique un second conduit 121 avec, soit le conduit d'entrée 100, soit le conduit d'admission 102, soit le conduit d'échappement 103. Par analogie, la troisième vanne rotative 112 peut, en fonction de la configuration déterminée par la seconde carte de commande 150, mettre en relation fluidique un troisième conduit 122 avec, soit le conduit d'entrée 100, soit le conduit d'admission 102, soit le conduit d'échappement 103.

Par ailleurs, comme illustré sur Fig. 3, le système de purification de gaz 1 comprend plusieurs adsorbeurs 130-132, aussi appelés cartouches d'adsorption, contenant chacun un ou des adsorbants servant à purifier le gaz expiré (i.e. mélange O₂/CO₂), de préférence 3 adsorbeurs 130-132 agencés en parallèle.

Plus précisément, agencée dans le premier conduit 120 se trouve une première cartouche ou adsorbeur 130, présentant un premier port amont 130a, i.e. un orifice d'entrée, et un premier port aval 130b, i.e. un orifice de sortie. De façon similaire, une seconde cartouche ou adsorbeur 131 se trouve agencée dans le second conduit 121 et présente un second port amont 131a et un second port aval 131b, et une troisième cartouche ou adsorbeur 132 se trouve agencée dans le troisième conduit 122 et présente un troisième port amont 132a et troisième port aval 131b.

Les première, seconde et troisième cartouches 130, 131, 132 sont des récipients d'adsorption agencées en parallèle sur le trajet du gaz véhiculé par les premier, second et troisième conduits 120-122, respectivement, à savoir le gaz expiré (i.e. mélange O₂/CO₂) devant être épuré pour produire de l'oxygène.

A cette fin, les cartouches 130-132 renferment chacune un ou plusieurs adsorbants, i.e. tamis moléculaire, de préférence agencés en lit de particules d'absorbant, par exemple un (ou des) adsorbant de type zéolite, telle une zéolite 13X, et/ou de type silicate. Par exemple, chaque cartouche 130-132 est dimensionnée pour contenir environ 500 g de particules de zéolite 13X et de silicates. Les particules d'adsorbant sont typiquement des billes, des extrudés ou analogues. Dans tous les cas, on choisit un (des) adsorbant permettant d'adsorber ou piéger préférentiellement le CO₂, voire aussi la vapeur d'eau, c'est-à-dire présentant une sélectivité plus élevée pour le CO₂ (et de préférence l'eau) que pour l'oxygène, de manière à produire un flux purifié contenant une concentration en oxygène supérieure à celle dans le gaz expiré par le patient, c'est-à-dire avant sa purification.

L'adsorbant permet de concentrer le flux en oxygène en éliminant la majorité, voire (quasi) totalité, des autres espèces qui s'y trouvent, typiquement le CO₂ et l'eau, et/ou d'autres impuretés inévitables.

Bien entendu, d'autres matériaux adsorbants, tels que des MOF (Metal Organic Framework) ou autres peuvent être utilisés en remplacement ou supplément pour augmenter la capacité et/ou sélectivité d'adsorption du CO₂.

Lorsque le flux de gaz (i.e. mélange O₂/CO₂) a traversé l'une ou l'autre des cartouches 130-132, il ressort sous forme de gaz purifié, à savoir un flux d'oxygène de haute pureté (>95% vol. env.), par l'un des orifices de sortie ou ports avals 130b-132b, avant d'être acheminé par l'un ou l'autre des premier, second et troisième conduits 120-122 jusqu'à respectivement une quatrième vanne rotative 140, une cinquième vanne rotative 141 et une sixième vanne rotative 142, lesquelles sont identiques ou similaires à la première vanne rotative 110.

L'unité de purification de gaz expiré 1 comprend en outre un conduit de sortie 107 résultant de la réunion (en 107a) de trois sous-conduits, à savoir un premier sous-conduit de sortie 1070, un second sous-conduit de sortie 1071 et un troisième sous-conduit de sortie 1072 qui sont respectivement reliés fluidiquement à la quatrième vanne rotative 140, à la cinquième vanne rotative 141 et à la sixième vanne rotative 112.

Le conduit de sortie 107 est raccordé fluidiquement à la ligne de recyclage de gaz 11 pour l'alimenter en oxygène produit par l'unité de purification de gaz expiré 1 et permettre son acheminement par ladite ligne de recyclage de gaz 11 jusqu'au réservoir-tapon 24, comme déjà expliqué.

En fait, le conduit d'entrée de gaz 100 et le conduit de sortie 107 font partie d'un circuit de recyclage de gaz 502 interne au système de purification de gaz 1 qui permet de véhiculer les flux de gaz, à savoir le flux de gaz expiré à purifier, i.e. mélange O₂/CO₂, et le flux de gaz purifié, i.e. O₂), depuis le conduit d'entrée de gaz 100 jusqu'au conduit de sortie 107.

Le conduit d'entrée 100 est alimenté en mélange O₂/CO₂ par la ligne de récupération de gaz 11, alors que le conduit de sortie 107 est raccordé fluidiquement à la ligne de recyclage de gaz pour l'alimenter en oxygène, i.e. en gaz recyclé, produit par l'unité de purification de gaz expiré 1.

Le circuit de recyclage de gaz 502 comprend des conduits d'air 100, 107, 120-122, et au moins un adsorbeur 130-132, de préférence les adsorbeurs 130-132, et des vannes de contrôle 110-112, 140-142, en particulier des vannes rotatives.

De plus, l'unité de purification de gaz expiré 1 comprend aussi un conduit d'extraction d'air 108 comprenant un port de sortie d'air 108c, relié au premier conduit d'interconnexion 31 de Fig. 2, via un système de raccordement comprenant par exemple des connecteurs réciproques de type mâle/femelle permettant d'assurer une connexion mécanique et fluidique.

Le conduit d'extraction 108 résulte, lui aussi, de la réunion (en 108a, 108b) de trois sous-conduits, à savoir un premier sous-conduit d'extraction 1080, un second sous-conduit d'extraction 1081 et un troisième sous-conduit d'extraction 1082, qui sont respectivement reliés fluidiquement, i.e. débouchent, à la quatrième vanne rotative 140, à la cinquième vanne rotative 141 et à la sixième vanne rotative 142.

Le conduit d'extraction 108 est relié fluidiquement, via le premier conduit d'interconnexion 31, au conduit d'admission 310 de l'unité de génération d'O₂ 3 pour lui fournir l'air qui est utilisé dans le module de séparation d'O₂ 316 pour produire de l'oxygène (quasi)pur.

Enfin, l'unité de purification de gaz expiré 1 comprend aussi un conduit de régénération 109 relié fluidiquement au second conduit d'interconnexion 33 de Fig. 2 qui est lui-même raccordé fluidiquement au conduit d'échappement 330 de l'unité de génération d'O₂ 3, de manière à pouvoir recueillir et acheminer le gaz-déchet provenant de l'unité de génération d'O₂ 3, comme expliqué ci-après.

Là encore, le conduit de régénération 109 résulte de la réunion (en 109a, 109b) de trois sous-conduits, à savoir un premier sous-conduit de régénération 1090, un second sous-conduit de régénération 1091 et un troisième sous-conduit de régénération 1092, qui sont respectivement reliés fluidiquement, i.e. débouchent, à la quatrième vanne rotative 140, à la cinquième vanne rotative 141 et à la sixième vanne rotative 142.

Etant donné que les différentes vannes rotatives et les différents conduits et sous-conduits agencés de part et d'autre des première, seconde et troisième cartouches 130, 131, 132 sont identiques, en fonction de la configuration déterminée par la seconde carte de commande 150 :
- la quatrième vanne rotative 140 peut mettre en relation fluidique le premier conduit 120 avec soit le conduit de sortie 107, soit le conduit d'extraction 108, soit le conduit de régénération 109.
- la cinquième vanne rotative 141 peut mettre en relation fluidique le second conduit 121 avec soit le conduit de sortie 107, soit le conduit d'extraction 108, soit le conduit de régénération 109.
- la sixième vanne rotative 142 peut mettre en relation fluidique le troisième conduit 122 avec soit le conduit de sortie 107, soit le conduit d'extraction 108, soit le conduit de régénération 109.

Le fonctionnement de l'unité d'épuration de gaz expiré 1 de l'installation de fourniture d'O2 illustrée en Fig. 1 est décrit ci-après, de manière séquentielle en configurant successivement les vannes de rotation de l'unité d'épuration de gaz expiré 1.

On considère que, dans la configuration initiale, c'est-à-dire fixée par la carte de commande 150 contrôlant les différentes vannes rotatives de l'unité d'épuration de gaz expiré 1 :
- la première vanne rotative 110 réalise une communication fluidique entre le conduit d'entrée 100 et le premier conduit 120. La quatrième vanne rotative 140 réalise une communication fluidique entre le premier conduit 120 et le conduit de sortie 107. Il existe ainsi une communication fluidique entre le conduit d'entrée 100 et le conduit de sortie 107.
- la second vanne rotative 111 réalise une communication fluidique entre le conduit d'admission 102 et le second conduit 121. La cinquième vanne rotative 141 réalise une communication fluidique entre le second conduit 121 et le conduit d'extraction 108. Il existe ainsi une communication fluidique entre le conduit d'admission 102 et le conduit d'extraction 108.
- la troisième vanne rotative 112 réalise une communication fluidique entre le conduit d'échappement 103 et le troisième conduit 122. La sixième vanne rotative 142 réalise une communication fluidique entre le troisième conduit 122 et le conduit de régénération 109. Il existe ainsi une communication fluidique entre le conduit d'échappement 103 et le conduit de régénération 109.

Le fonctionnement de l'unité d'épuration de gaz expiré 1 est détaillé en lien avec d'abord la première cartouche 130, puis les deuxième et troisième cartouches 131, 132.

Comme déjà dit, le gaz expiré par le patient P (i.e. mélange O₂/CO₂), qui contient une haute teneur en O₂, par exemple de l'ordre de 90% à 95% en vol., mais aussi du CO₂ (env. 5% vol) et généralement de la vapeur d'eau, emprunte, la ligne de récupération de gaz 10 formant un circuit expiratoire, avant d'être admis dans l'unité d'épuration de gaz expiré 1 de Fig. 3, via le conduit d'entrée 100.

Du fait des configurations pneumatiques imposées par la carte de commande 150, le conduit d'entrée 100 est fluidiquement relié au conduit de sortie 107 via le premier conduit 120, sur lequel est agencé la première cartouche 130 ou adsorbeur. Ainsi, le gaz empruntant le conduit d'entrée 100 est dirigé dans la première cartouche 130, via son premier port amont 130a, pour y être débarrassé du CO₂ et préférentiellement de la vapeur d'eau qu'il contient, c'est-à-dire que le ou les adsorbants remplissant la première cartouche ou adsorbeur 130 captent, c'est-à-dire retiennent/piègent les molécules de CO₂ et préférentiellement de vapeur d'eau, en laissant passer l'O₂, c'est-à-dire sans l'adsorber.

Par exemple, la capacité et/ou la sélectivité d'adsorption du CO₂ et de la vapeur d'eau des zéolites, en particulier la zéolite 13X, ou bien des silicates est supérieure à la capacité/sélectivité d'adsorption de l'O₂ de sorte que ces composés CO₂ et vapeur d'eau soient préférentiellement piégés/adsorbés, alors que l'oxygène peut traverser librement le lit d'adsorbant sans y être retenu ou très peu.

Ainsi, le gaz purifié sortant de la première cartouche 130, via le premier port aval, 130b est épuré du CO₂ et de la vapeur d'eau, est un flux d'oxygène contenant essentiellement de l'oxygène, à savoir formé typiquement de plus de 99.5% d'oxygène.

Après sa sortie de l'unité d'épuration de gaz expiré 1, il est récupéré et acheminé par la ligne de recyclage de gaz 11 formant un circuit d'échappement, en ayant au préalable transité dans le premier conduit 120 situé en aval de la première cartouche 130 et le conduit de sortie 107, comme déjà expliqué.

Le gaz purifié peut être alors réinjecté dans la ligne principale d'acheminement de gaz 23, via le réservoir 24 dans lequel il pénètre via le port additionnel 12. Ce flux de gaz purifié qui contient de l'oxygène à haute concentration (e.g. >99.5%) se mélange donc, au sein du réservoir 24, avec de l'oxygène (e.g. 100%) provenant de la source d'oxygène 3 de manière à obtenir un mélange d'oxygène de très haute pureté (i.e. proche de 100% vol), qui peut être ensuite acheminé jusqu'au patient P et lui être administré via l'interface respiratoire 21 afin d'être ré-inhalé. Autrement dit, le flux de gaz purifié vient se substituer à une partie de l'oxygène provenant de la source d'oxygène 3, ce qui permet de limiter la consommation d'oxygène provenant de ladite source d'oxygène 3 et par ailleurs le gaspillage d'oxygène en recyclant le gaz expiré qui serait normalement rejeté à l'atmosphère.

Le système de purification de gaz 1 de Fig. 3 de l'installation de fourniture 50 de gaz selon l'invention de Fig. 1 fonctionne de manière cyclique, du fait de l'utilisation d'adsorbant(s) dans les adsorbeurs 130-132 qui nécessite d'être régénéré afin de désorber/libérer les composés ayant été piégés, à savoir le CO₂ et la vapeur d'eau issus des phases expiratoires du patient.

En effet, l'adsorbant (ou les adsorbants) possède une capacité maximale d'adsorption au-delà de laquelle l'adsorbant est saturé, c'est-à-dire ne peut plus capter les molécules de CO₂, voire celles d'eau, et ces molécules peuvent alors traverser l'adsorbant avec l'oxygène produit et se retrouver dans le gaz « purifié », ce qui n'est pas souhaitable.

Afin d'éviter une telle situation de saturation de l'adsorbant, et que le patient P ne puisse ré-inhaler du CO₂ (avec l'oxygène) et donc éviter une situation dangereuse pour celui-ci, il convient de régénérer périodiquement chaque adsorbant afin de le débarrasser des composés qui y sont adsorbés/retenus, en particulier le CO₂.

Selon un mode de réalisation, la capacité maximale d'adsorption de la première cartouche 130, c'est-à-dire le volume maximal de CO₂ pouvant y être retenu, i.e. capté, est connue et mémorisée au sein du microcontrôleur 151 ou ailleurs. En mesurant le volume total de gaz expiré ayant été admis dans l'unité d'épuration des gaz expirés 1, au moyen du premier capteur de débit 101, et en considérant une concentration de CO₂ de l'ordre de 5% vol., le microcontrôleur 151 est en mesure de déterminer que la première cartouche 130 est proche d'arriver à saturation. On procède de la même manière pour les autres cartouches 131, 132, i.e. les autres adsorbeurs.

Bien entendu, selon d'autres modes de réalisation, l'évaluation du taux de saturation des cartouches 130-132, i.e. des adsorbeurs, pourrait être opérée autrement, par exemple en mesurant la température des adsorbants ou encore en intégrant une mesure de teneur en CO₂ dans le conduit de sortie 107. Selon encore un autre mode de réalisation, la régénération des cartouches 130-132 pourrait aussi être opérée après une durée donnée ou de toute autre façon.

Lorsque la première cartouche 130, i.e. le premier adsorbeur, ou une autre cartouche 131, 132, parvient à saturation, le microcontrôleur 151 commande la seconde vanne rotative 111 et la cinquième vanne rotative 141 afin de basculer sur une cartouche non saturée et/ou ayant été régénérée, afin de permettre au système de continuer à purifier les gaz expirés par le patient P, pendant que la cartouche ou adsorbeur saturé est régénéré.

La seconde vanne rotative 111 réalise une connexion fluidique entre le conduit d'entrée 100 et le second conduit 121, alors que, dans le même temps, la cinquième vanne rotative 141 réalise une connexion fluidique entre le même second conduit 121, en particulier sa portion aval située en aval du second port aval 131b de la seconde cartouche 131, et le conduit de sortie 107. Dans le même temps, le microcontrôleur 151 commande la première vanne rotative 110 et la quatrième vanne rotative 140 de manière à mettre en relation fluidique respectivement le conduit d'échappement 103 et le premier conduit 120, ainsi que la portion aval du premier conduit 120, i.e. en aval du premier port aval 130b de la première cartouche 130, et le conduit de régénération 109.

Le microcontrôleur 151 commande par ailleurs la troisième vanne rotative 112 et la sixième vanne rotative 142 de manière à mettre en relation fluidique respectivement le conduit d'admission 102 et le troisième conduit 122, ainsi que la portion aval dudit troisième conduit 122 et le conduit d'extraction 108.

Ainsi, lorsque la première cartouche 130 est saturée, elle doit être régénérée. Classiquement, lors d'une régénération d'un lit d'adsorbant, un gaz sec, c'est-à-dire dépourvu d'humidité, i.e. de vapeur d'eau, par exemple du N₂ issu d'une bouteille, est chauffé à haute température, par exemple supérieure à 250°C et traverse l'adsorbant. En gérant de façon appropriée le profil de température, il est possible de régénérer l'adsorbant.

Toutefois, dans le cadre de la présente invention, une régénération de ce type n'est pas adaptée du fait de l'absence, de toute source de gaz sec sur site d'utilisation, typiquement en hôpital ou analogue. De plus, le fait de devoir porter le gaz à haute température entraîne une complexité technique et donc induit un surcoût ainsi qu'une consommation électrique importante.

Dans le cadre de l'invention, on utilise le flux de gaz-déchet provenant de l'unité de génération d'O₂ 3 pour opérer la régénération.

En effet, on comprend, au vu de Fig. 2, que le conduit d'échappement 330 de l'unité de génération d'O₂ 3 convoie un gaz appauvri en O₂ (i.e. de concentration inférieure à 21% vol.), typiquement <19% O₂, à haute température, c'est-à-dire à au moins 300°C environ.

Le conduit d'échappement 330 est fluidiquement relié au second conduit d'interconnexion 33 et au conduit de régénération 109 de l'unité d'épuration de gaz expiré 1, le flux de gaz-déchet provenant de l'unité de génération d'O₂ 3 est amené jusqu'au conduit de régénération109 et est dirigé ensuite dans le premier conduit 120 de la première cartouche 130, du fait de la configuration pneumatique de la quatrième vanne rotative 140.

Ce gaz-déchet va alors être utilisé comme gaz de régénération étant donné qu'il va pénétrer la première cartouche 130 via le premier port aval 130b, désorber une partie du CO₂ et de la vapeur d'eau adsorbés sur l'adsorbant contenu dans la première cartouche 130 et en sortir par le premier port amont 130a en entraînant avec lui les composés désorbés, i.e. CO₂ et éventuellement H₂O.

En d'autres termes, le flux de régénération sortant de la première cartouche 130 via le premier port amont 130a, est formé du gaz-déchet enrichi en CO₂ et préférentiellement en vapeur d'eau.

Bien que ce gaz-déchet servant de gaz de régénération, circulant dans le conduit de régénération 109, contienne des traces (i.e. très faibles teneurs) de vapeur d'eau et de CO₂, puisqu'il émane de l'air ambiant ayant été fourni à l'unité de génération d'O₂ 3 pour produire de l'oxygène, la température élevée de ce gaz-déchet, i.e. d'au moins 300°C environ, permet d'assurer une désorption complète de la première cartouche 130 sans entrainer par ailleurs d'adsorption temporaire de ces traces de vapeur d'eau et de CO₂.

Le débit gazeux circulant dans la portion amont du premier conduit 120 arrive alors sur la première vanne rotative 110 et emprunte, du fait de la configuration pneumatique de ladite première vanne rotative 110, le conduit d'échappement 103, pour être évacué à l'air ambiant via le port de sortie 103c du conduit d'échappement 103.

Autrement dit, on obtient une régénération par le flux de gaz-déchet chauffé laquelle est opérée à contre-courant, c'est-à-dire dans le sens opposé au sens de circulation du gaz pendant l'étape d'adsorption, c'est-à-dire de production d'oxygène.

Le flux de régénération sortant de l'adsorbeur en cours de régénération est évacué à l'atmosphère par le conduit d'échappement 103.

Autrement dit, le système de purification de gaz 1 comprend un circuit de régénération 501 interne servant à acheminer le gaz-déchet qui lui est fourni par l'unité de génération d'oxygène 50, via le second conduit d'interconnexion 33, depuis le conduit de régénération 109 jusqu'au conduit d'échappement 103 relié à l'atmosphère afin de permettre d'y évacuer le flux gazeux de régénération, après sa traversé d'au moins un adsorbeur 130-132. Le circuit de régénération 501 comprend donc des passages ou conduits d'air103, 109, 120-122 et au moins un adsorbeur, de préférence les adsorbeurs 130-132, et aussi des vannes de contrôle, en particulier les vannes rotatives 110-112, 140-142.

Pendant que la première cartouche 130 est en phase de régénération, le gaz empruntant le conduit d'entrée 100 est dirigé dans la seconde cartouche 131, via son second port amont 131a, pour y être débarrassé du CO₂ et de la vapeur d'eau et produire un flux d'oxygène, comme déjà expliqué, sortant de la seconde cartouche 131, via le second port aval, 130b en ayant une teneur en oxygène généralement de plus de 99.5% vol.

Après sa sortie de l'unité de purification de gaz expiré 1, il est récupéré et acheminé par la ligne de recyclage de gaz 11 en ayant au préalable transité dans le second conduit 121 situé en aval de la seconde cartouche 131 et le conduit de sortie 107. Ce gaz purifié est alors réinjecté dans la ligne principale d'acheminement de gaz 23, via le réservoir-tampon 24, comme déjà détaillé.

Comme précédemment, le microcontrôleur 151 peut alors déterminer que la seconde cartouche 131 parvient également à saturation et commande alors la troisième vanne rotative 112 et la sixième vanne rotative 142 afin de basculer sur une cartouche non saturée et/ou ayant été régénérée, permettant de continuer à épurer les gaz expirés par le patient P.

A ce moment, la troisième vanne rotative 112 réalise une connexion fluidique entre le conduit d'entrée 100 et le troisième conduit 122, alors que dans le même temps, la sixième vanne rotative 142 réalise une connexion fluidique entre le même troisième conduit 122, en particulier sa portion aval située en aval du troisième port aval 132b de la troisième cartouche 132, et le conduit de sortie 107.

Dans le même temps, le microcontrôleur 151 commande la première vanne rotative 110 et la quatrième vanne rotative 140 de manière à mettre en relation fluidique respectivement le conduit d'admission 102 et le premier conduit 120, ainsi que la portion aval du premier conduit 120, i.e. en aval du premier port aval 130b de la première cartouche 130, et le conduit d'extraction 108 de manière à permettre à de l'air ambiant de pénétrer dans le système de purification 1 via le conduit d'admission 102 et en ressortir via le conduit d'extraction 108 qui alimente le conduit d'interconnexion 31 amenant le flux d'air, via le conduit d'admission 310, à l'unité de génération d'oxygène 30, où il est séparé au sein d'un (ou plusieurs) module de séparation électrochimique 316 afin de produire le flux d'oxygène de haute pureté et par ailleurs le flux de gaz-déchet servant à la régénération des adsorbants, comme déjà expliqué.

En fait, l'air est acheminé au sein de l'unité d'épuration de gaz expiré 1 dans un circuit d'air 500 interne comprenant des conduits ou passages d'air, en particulier le conduit d'admission 102, le conduit d'extraction 108 et tous les conduits de liaison intermédiaires 120-122, et des éléments agencés sur ces conduits 120-122, en particulier le ou les adsorbeurs 130-132 et une ou des vannes de contrôle, en particulier les vannes rotatives 110-112 ; 140-142.

L'air circulant dans le circuit d'air 500 traverse donc au moins un adsorbeur 130-132 avant d'être évacué par le conduit d'extraction 108 et envoyé vers l'unité de génération d'oxygène 30 via le premier conduit d'interconnexion 31.

Ceci est avantageux car le flux d'air prélevé à l'atmosphère est à température ambiante, c'est-à-dire de l'ordre de 15 à 30°C en général. Il peut donc refroidir l'adsorbant(s) ayant été régénéré par le gaz-déchet chaud.

Bien que le flux d'air puisse comprendre du CO₂ atmosphérique en tant qu'impureté, la quantité de CO₂ qui s'y trouve est négligeable (max. env. 0.005% vol.) comparée à celle présente dans le gaz expiré à purifier (i.e. env. 5% vol.) et n'impacte pas les performances du ou des adsorbants.

De même, la quantité de vapeur d'eau (i.e. humidité) pouvant se trouver dans le flux d'air en tant qu'impureté est aussi faible et, dans tous les cas, insuffisante pour impacter négativement, i.e. de manière significative, la capacité d'adsorption globale du ou des adsorbants.

De toute façon, on peut dimensionner les adsorbeurs et la quantité d'adsorbant qui s'y trouve pour prendre en compte des impuretés atmosphériques et leur influence négative potentielle sur le ou les adsorbants.

Alternativement, on peut prévoir un lit d'adsorbant dédié supplémentaire pour piéger ces impuretés.

Le microcontrôleur 151 commande par ailleurs la deuxième vanne rotative 111 et la cinquième vanne rotative 141 de manière à mettre en relation fluidique respectivement le conduit d'échappement 103 et le second conduit 121, ainsi que la portion aval du second conduit 122 et le conduit de régénération 109.

Le gaz expiré (i.e. mélange O₂/CO₂) empruntant le conduit d'entrée 100 est alors dirigé dans la troisième cartouche 132, via le troisième port amont 131a, pour y être débarrassé du CO₂ et préférentiellement de la vapeur d'eau et sortir de la troisième cartouche 131 pour emprunter le conduit de sortie 107 par le troisième conduit 122.

Là encore, après sa sortie de l'unité de purification de gaz expiré 1, le flux de gaz formé d'oxygène est récupéré et acheminé au patient P par la ligne de recyclage de gaz 11 et les lignes d'acheminement successives.

Dans le même temps, le gaz-déchet à haute température circulant dans le conduit de régénération 109, est dirigé dans le second conduit 121 de la seconde cartouche 131, du fait de la configuration pneumatique de la cinquième vanne rotative 141. Ce gaz-déchet va y pénétrer, via le second port aval 131b et entrainer une partie du CO₂ et de la vapeur d'eau que la cartouche 131 a piégé sur son adsorbant, pour en sortir via le second port amont 131a. Le débit se propageant dans la portion amont du second conduit 121 débouche alors sur la seconde vanne rotative 111 et emprunte, du fait de la configuration pneumatique de celle-ci, le conduit d'échappement 103, pour être évacué à l'atmosphère ambiante via le port de sortie 103c du conduit d'échappement 103.

Dans l'installation de fourniture d'O₂ 50 de Fig. 1, le conduit d'admission 310 de l'unité de génération d'O₂ 3 de Fig. 2 est relié au conduit d'extraction 108 de l'unité d'épuration de gaz expiré 1 de Fig. 3, via le premier conduit d'interconnexion 31, afin de pouvoir alimenter l'unité de génération d'O₂ 3, en particulier la première ligne de gaz interne 3100 avec de l'air provenant de l'unité d'épuration de gaz expiré 1.

Au sein de l'unité d'épuration de gaz expiré 1, le contrôle de la circulation de l'air au sein du circuit d'air 500 interne allant du conduit d'admission 102 au conduit d'extraction 108 est opéré comme suit, en prenant l'exemple d'un transit via le premier adsorbeur 130, sachant qu'un contrôle analogue est opéré lorsque l'air traverse les autres adsorbeurs.

La quatrième vanne rotative 140 est commandée par les moyens de pilotage 150, 151, pour réaliser une connexion fluidique entre la portion aval du premier conduit 120, i.e. en aval du premier port aval 130b de la première cartouche 130, et le conduit d'extraction 108, alors que la première vanne rotative 110 est commandée par les moyens de pilotage 150, 151, pour réaliser une connexion fluidique entre le conduit d'admission 102 et le premier conduit 120. Il existe alors une communication fluidique entre le conduit d'admission 102 et le conduit d'extraction 108 via respectivement le premier conduit 120 et première cartouche 130, permettant à l'air de circuler du conduit d'admission 102 au conduit d'extraction 108 grâce à la force d'aspiration opérée par les moyens d'aspiration de gaz 311, tel un ventilateur ou similaire, agencés dans la première ligne de gaz interne 3100 de l'unité d'épuration de gaz expiré 1.

En effet, comme le conduit d'admission 102 est relié à l'air ambiant via son port 102c, on peut alors considérer que les moyens d'aspiration de gaz 311, tel un ventilateur, agencés dans le conduit d'admission 310 de l'unité de génération d'O₂ 3 peuvent aspirer de l'air ambiant à travers le port 102c, et que cet air aspiré transite alors, respectivement, par le conduit d'admission 102, le premier conduit 120 sur lequel est agencé la première cartouche 130 et le conduit d'extraction 108 de l'unité d'épuration de gaz expiré 1, pour ensuite être acheminé par le premier conduit d'interconnexion 31 jusqu'au conduit d'admission 31 de l'unité de génération d'O₂ 3.

Autrement dit, l'unité de génération d'O₂ 3 est alimentée en air par le conduit d'interconnexion 31 qui vient se raccorder fluidiquement au conduit d'admission 310 par l'une de ses extrémités, et au conduit 108, par son autre extrémité, lui-même relié au conduit d'admission 102 en communication avec l'atmosphère via l'orifice 102c.

Le flux d'air aspiré étant à température ambiante, typiquement entre 15 et 30°C, il va refroidir le premier adsorbeur 130 en le traversant, en particulier l'adsorbant qui s'y trouve, sachant qu'il a été précédemment chauffé par le gaz-déchet chaud ayant servi à la régénération de cet adsorbeur 130 pendant une étape de régénération précédente.

Ainsi, on utilise de façon avantageuse l'unité de génération d'O₂ 3 pour aspirer de l'air servant à refroidir la première cartouche ou adsorbeur 130, pour permettre à son adsorbant de revenir à température ambiante avant d'être réutilisé pour capter le CO₂ et la vapeur d'eau contenue dans les gaz expirés, lors d'une phase de purification suivante.

On comprend que l'unité d'épuration de gaz expiré 1 est commandée de manière cyclique afin d'assurer en permanence sa fonction d'épuration des gaz expirés tout en assurant la régénération et le reconditionnement en température, i.e. refroidissement, des cartouches d'adsorption 130-132.

Dans l'exemple ci-dessus, lorsque la première cartouche 130 est saturée, la seconde cartouche 131 devient la cartouche active permettant d'épurer les gaz expirés, alors que la première cartouche 130 entame sa phase de régénération. Ensuite, lorsque la seconde cartouche 131 devient saturée, la troisième cartouche 132 devient la cartouche active et la seconde cartouche 131 entame une phase de régénération alors que la première cartouche 130 entame une phase de refroidissement. Lorsque la troisième cartouche 132 devient saturée, la première cartouche 130 redevient alors la cartouche active, tandis que la troisième cartouche entame 132 une phase de régénération et la seconde cartouche 131 entame une phase de refroidissement.

Une telle séquence se répète de façon cyclique pendant le fonctionnement de l'installation 50.

Selon les conditions et paramètres de fonctionnement et l'architecture de l'installation 50, tel que dimensionnement des cartouches, nature des adsorbants, température de régénération..., les durées ou temps respectifs pour arriver à la saturation, à la complète régénération et au refroidissement à température ambiante des différentes cartouches d'adsorption peuvent être équivalents ou égaux, ou différents.

En fonction des disparités observées entre les différents temps de saturation, de régénération et de refroidissement, un nombre additionnel de cartouches, associées à leurs propres vannes rotatives, connectées via des sous-conduits additionnels aux conduits décrits ci-dessus peut être intégré dans l'unité de purification de gaz expiré 1. Par exemple, si le temps pour régénérer et refroidir une cartouche est le double du temps nécessaire à sa limite de saturation, l'unité de purification de gaz expiré 1 peut intégrer 5 cartouches afin de doubler les temps de régénération et de refroidissement une fois qu'une cartouche est arrivée à saturation.

L'installation de fourniture de gaz respiratoire 50 de l'invention est adaptée à l'administration de gaz à un patient en état hypoxémique, notamment une personne en état hypoxémique infectée par un coronavirus, tel le Covid-19 ou analogue, que ce soit un adulte, notamment une personne âgée, un adolescent ou enfant.

Une telle installation de fourniture de gaz 50 constitue un ensemble de fourniture d'oxygène mettant en œuvre une source « sur site » d'oxygène gazeux permettant de délivrer un flux d'alimentation riche en oxygène gazeux, tel de l'oxygène pur ou quasiment pur (i.e. 99.5% à 100%vol.), d'une part, et un système d'épuration des gaz expirés, d'autre part, pour récupérer l'oxygène, normalement perdu, qui est présent dans les gaz expirés par le patient (en mélange avec du CO₂), puis le recycler ensuite dans le flux d'alimentation riche en oxygène, et ainsi assurer une délivrance d'oxygène à haute concentration d'O₂ (>95 à 99%vol) à un utilisateur, typiquement un patient.

Ceci permet de limiter la consommation globale d'O₂ tout en garantissant la fourniture d'une haute concentration d'O₂. Autrement dit, grâce à l'installation de fourniture de gaz de l'invention, on peut assurer une FiO₂ supérieure à 90% chez le patient sans avoir recours à un débit élevé d'oxygène provenant de la source d'oxygène étant donné qu'une proportion non-négligeable du flux d'oxygène fourni au patient se compose d'oxygène ayant été purifié au sein du système de purification de gaz puis recyclé dans la ligne principale d'acheminement de gaz, via la ligne de recyclage de gaz. Réutiliser une partie de l'oxygène normalement rejeté à l'atmosphère permet de réduire la consommation globale d'oxygène et donc de limiter fortement le stress sur les réserves d'O₂ disponibles sur le site considéré, i.e. hôpital ou autre, en limitant les pertes d'oxygène.

Par ailleurs, utiliser un (ou des) module de séparation électrochimique 316 à membrane céramique, de préférence dopée, présente l'avantage de fournir de l'oxygène très haute pureté, condition nécessaire pour permettre à l'installation de fourniture de gaz de la présente invention de recycler les gaz expirés par le patient.

## Revendications

1. Installation de fourniture (50) de gaz respiratoire à un utilisateur (P) comprenant :
- une source de gaz (3) pour fournir un gaz respiratoire contenant de l'oxygène,
- une ligne principale d'acheminement de gaz (23) reliant fluidiquement la source de gaz (3) à une interface respiratoire (21) configurée pour administrer le gaz respiratoire contenant de l'oxygène à l'utilisateur, lors de chaque phase inspiratoire dudit utilisateur,
- une ligne de récupération de gaz (10) configurée pour récupérer et acheminer au moins une partie du mélange gazeux CO₂/O₂ expiré par l'utilisateur et se retrouvant dans l'interface respiratoire, lors de chaque phase expiratoire de l'utilisateur, ladite ligne de récupération de gaz (10) reliant fluidiquement l'interface respiratoire (21) à un système de purification de gaz (1) comprenant des adsorbeurs (130-132) agencés en parallèle contenant chacun au moins un adsorbant, ladite ligne de récupération de gaz (10) alimentant ledit système de purification de gaz (1) en ledit mélange gazeux CO₂/O₂ expiré par l'utilisateur et
- une ligne de recyclage de gaz (11) reliant fluidiquement le système de purification de gaz (1) à la ligne principale d'acheminement de gaz (23),
**caractérisée en ce que** la source de gaz (3) comprend une unité de génération d'oxygène (30) comprenant :
- au moins un module de séparation électrochimique (316),
- une première ligne de gaz (3100) pour alimenter ledit audit au moins un module de séparation électrochimique (316) avec de l'air,
- des moyens de chauffage de gaz (313) agencés sur la première ligne de gaz interne (3100), en amont dudit au moins un module de séparation électrochimique (316) et
- une seconde ligne de gaz (3200) reliant fluidiquement ledit au moins un module de séparation électrochimique (316) au système de purification de gaz (1), pour convoyer un flux de gaz-déchet jusqu'au système de purification de gaz (1) pour régénérer ledit au moins un adsorbant contenu dans au moins un desdits adsorbeurs (130-132).

2. Installation selon la revendication 1, **caractérisée en ce que** le système de purification de gaz (1) comprend au moins trois adsorbeurs (130-132) agencés en parallèle.

3. Installation selon la revendication 1, **caractérisée en ce que** la ligne principale d'acheminement de gaz (23) comprend un réservoir-tampon (24) et la ligne de recyclage de gaz (11) est raccordée fluidiquement au réservoir-tampon (24).

4. Installation selon la revendication 1, **caractérisée en ce que** le système de purification de gaz (1) est relié fluidiquement à la première ligne de gaz (3100) de l'unité de génération d'oxygène (30) pour fournir de l'air à ladite première ligne de gaz (3100), de préférence via un premier conduit d'interconnexion (31).

5. Installation selon la revendication 4, **caractérisée en ce que** le système de purification de gaz (1) comprend un circuit d'air (500) pour acheminer de l'air fourni à la première ligne de gaz (3100) de l'unité de génération d'oxygène (30).

6. Installation selon la revendication 5, **caractérisée en ce que** le circuit d'air (500) comprend des passages ou conduits d'air (102,108, 120-122), et au moins un adsorbeur (130-132), de préférence il comprend en outre au moins une vanne de contrôle (110-112 ; 140-142).

7. Installation selon la revendication 1, **caractérisée en ce que** ledit au moins un module de séparation électrochimique (316) de l'unité de génération d'oxygène (30) comprend une ou plusieurs membranes céramiques.

8. Installation selon l'une des revendications 1, 4 ou 5, **caractérisée en ce que** la première ligne de gaz (3100) de l'unité de génération d'oxygène (30) comprend des moyens d'aspiration de gaz (311), lesdits moyens de chauffage de gaz (313) étant agencés entre les moyens d'aspiration de gaz (311) et ledit au moins un module de séparation électrochimique (316).

9. Installation selon l'une des revendications 1 ou 8, **caractérisée en ce que** des moyens d'échange thermique (312) sont agencés sur la première ligne de gaz interne (3100), de préférence entre les moyens d'aspiration de gaz (311) et les moyens de chauffage de gaz (313).

10. Installation selon la revendication 1, **caractérisée en ce que** la seconde ligne de gaz (3200) convoie un gaz-déchet ayant une concentration en oxygène inférieure à 21% vol., typiquement inférieure à 20% vol ., et/ou à une température d'au moins 250°C environ, de préférence au moins 300°C environ.

11. Installation selon les revendications 1 et 9, **caractérisée en ce que** la seconde ligne de gaz (3200) est agencée en parallèle de la première ligne de gaz (3100) au sein desdits moyens d'échange thermique (312) de manière à opérer un échange thermique entre le flux d'air circulant dans la première ligne de gaz (3100) et le gaz-déchet circulant dans la seconde ligne de gaz (3200), de préférence un échange thermique à contre-courant.

12. Installation selon l'une des revendications 1 ou 6, **caractérisée en ce que** :
- l'unité de génération d'O₂ (30) comprend des premiers moyens de pilotage (350, 351) configurés pour piloter ledit au moins un module de séparation électrochimique (316) et/ou
- le système de purification de gaz (1) comprend des seconds moyens de pilotage (150, 151) pour piloter au moins une partie des vannes de contrôles (110-112 ; 140-142).

13. Installation selon la revendication 1, **caractérisée en ce que** l'interface respiratoire (21) est un masque respiratoire.

14. Installation selon la revendication 1, **caractérisée en ce qu'**un second conduit d'interconnexion (33) relie fluidiquement la seconde ligne de gaz (3200) de l'unité de génération d'O₂ (30) au système de purification de gaz (1).

15. Installation selon la revendication 1, **caractérisée en ce que** le système de purification de gaz (1) comprend en outre :
- un circuit de recyclage de gaz (502) pour véhiculer au moins le gaz expiré fourni par la ligne de récupération de gaz (10) et
- un circuit de régénération (501) pour véhiculer au moins le gaz-déchet fourni par l'unité de génération d'oxygène (50), lesdits circuit de recyclage de gaz (502) et circuit de régénération (501) comprenant des conduits de gaz et les adsorbeurs (130-132).

## Patentansprüche

1. Anlage (50) zur Bereitstellung von Atemgas für einen Benutzer (P), umfassend:
- eine Gasquelle (3), um ein Sauerstoff enthaltendes Atemgas bereitzustellen,
- eine Gastransporthauptleitung (23), welche die Gasquelle (3) fluidisch mit einer Atemschnittstelle (21) verbindet, die dazu ausgestaltet ist, dem Benutzer das Sauerstoff enthaltende Atemgas während jeder Einatemphase des Benutzers zu verabreichen,
- eine Gasrückgewinnungsleitung (10), die dazu ausgestaltet ist, mindestens einen Teil des von dem Benutzer während jeder Ausatemphase des Benutzers ausgeatmeten und sich in der Atemschnittstelle befindenden CO₂/O₂-Gasgemisches zurückzugewinnen und zu transportieren, wobei die Gasrückgewinnungsleitung (10) die Atemschnittstelle (21) fluidisch mit einem Gasreinigungssystem (1) verbindet, das parallel angeordnete Adsorber (130-132) umfasst, die jeweils mindestens ein Adsorbens enthalten, wobei die Gasrückgewinnungsleitung (10) das Gasreinigungssystem (1) mit dem von dem Benutzer ausgeatmeten CO₂/O₂-Gasgemisch versorgt, und
- eine Gasrückführungsleitung (11), die das Gasreinigungssystem (1) fluidisch mit der Gastransporthauptleitung (23) verbindet, **dadurch gekennzeichnet, dass** die Gasquelle (3) eine Sauerstofferzeugungseinheit (30) umfasst, umfassend:
- mindestens ein elektrochemisches Trennmodul (316),
- eine erste Gasleitung (3100), um das mindestens eine elektrochemische Trennmodul (316) mit Luft zu versorgen,
- Gasheizmittel (313), die an der ersten internen Gasleitung (3100) stromauf des mindestens einen elektrochemischen Trennmoduls (316) angeordnet sind, und
- eine zweite Gasleitung (3200), die das mindestens eine elektrochemische Trennmodul (316) fluidisch mit dem Gasreinigungssystem (1) verbindet, um einen Abgasstrom bis zu dem Gasreinigungssystem (1) zu leiten, um das in mindestens einem der Adsorber (130-132) enthaltene mindestens eine Adsorbens zu regenerieren.

2. Anlage nach Anspruch 1, **dadurch gekennzeichnet, dass** das Gasreinigungssystem (1) mindestens drei parallel angeordnete Adsorber (130-132) umfasst.

3. Anlage nach Anspruch 1, **dadurch gekennzeichnet, dass** die Gastransporthauptleitung (23) einen Pufferbehälter (24) umfasst und die Gasrückführungsleitung (11) fluidisch an den Pufferbehälter (24) angeschlossen ist.

4. Anlage nach Anspruch 1, **dadurch gekennzeichnet, dass** das Gasreinigungssystem (1) fluidisch mit der ersten Gasleitung (3100) der Sauerstofferzeugungseinheit (30) verbunden ist, um der ersten Gasleitung (3100) Sauerstoff bereitzustellen, bevorzugt über einen ersten Verbindungskanal (31).

5. Anlage nach Anspruch 4, **dadurch gekennzeichnet, dass** das Gasreinigungssystem (1) einen Luftkreislauf (500) umfasst, um der ersten Gasleitung (3100) der Sauerstofferzeugungseinheit (30) bereitgestellte Luft zu transportieren.

6. Anlage nach Anspruch 5, **dadurch gekennzeichnet, dass** der Luftkreislauf (500) Luftdurchlässe oder -kanäle (102, 108, 120-122) umfasst, und mindestens einen Adsorber (130-132), er bevorzugt ferner mindestens ein Steuerventil (110-112; 140-142) umfasst.

7. Anlage nach Anspruch 1, **dadurch gekennzeichnet, dass** das mindestens eine elektrochemische Trennmodul (316) der Sauerstofferzeugungseinheit (30) eine oder mehrere keramische Membranen umfasst.

8. Anlage nach einem der Ansprüche 1, 4 oder 5, **dadurch gekennzeichnet, dass** die erste Gasleitung (3100) der Sauerstofferzeugungseinheit (30) Gasansaugmittel (311) umfasst, wobei die Gasheizmittel (313) zwischen den Gasansaugmitteln (311) und dem mindestens einen elektrochemischen Trennmodul (316) angeordnet sind.

9. Anlage nach einem der Ansprüche 1 oder 8, **dadurch gekennzeichnet, dass** Mittel zum thermischen Austausch (312) an der ersten internen Gasleitung (3100) angeordnet sind, bevorzugt zwischen den Gasansaugmitteln (311) und den Gasheizmitteln (313).

10. Anlage nach Anspruch 1, **dadurch gekennzeichnet, dass** die zweite Gasleitung (3200) ein Abgas leitet, das eine Sauerstoffkonzentration unter 21 Vol.-%, typischerweise unter 20 Vol.-% hat, und/oder mit einer Temperatur von mindestens etwa 250 °C, bevorzugt mindestens etwa 300 °C.

11. Anlage nach den Ansprüchen 1 und 9, **dadurch gekennzeichnet, dass** die zweite Gasleitung (3200) parallel zu der ersten Gasleitung (3100) innerhalb der Mittel zum thermischen Austausch (312) angeordnet ist, so dass ein thermischer Austausch zwischen dem in der ersten Gasleitung (3100) zirkulierenden Luftstrom und dem in der zweiten Gasleitung (3200) zirkulierenden Abgas, bevorzugt ein thermischer Austausch im Gegenstrom, vorgenommen wird.

12. Anlage nach einem der Ansprüche 1 oder 6, **dadurch gekennzeichnet, dass**:
- die O₂-Erzeugungseinheit (30) erste Ansteuerungsmittel (350, 351) umfasst, die dazu ausgestaltet sind, das mindestens eine elektrochemische Trennmodul (316) anzusteuern, und/oder
- das Gasreinigungssystem (1) zweite Ansteuerungsmittel (150, 151) umfasst, um mindestens einen Teil der Steuerventile (110-112; 140-142) anzusteuern.

13. Anlage nach Anspruch 1, **dadurch gekennzeichnet, dass** die Atemschnittstelle (21) eine Atemmaske ist.

14. Anlage nach Anspruch 1, **dadurch gekennzeichnet, dass** ein zweiter Verbindungskanal (33) die zweite Gasleitung (3200) der O₂-Erzeugungseinheit (30) fluidisch mit dem Gasreinigungssystem (1) verbindet.

15. Anlage nach Anspruch 1, **dadurch gekennzeichnet, dass** das Gasreinigungssystem (1) ferner umfasst:
- einen Gasrückführungskreislauf (502), um mindestens das von der Gasrückgewinnungsleitung (10) bereitgestellte ausgeatmete Gas zu befördern, und
- einen Regenerierungskreislauf (501), um mindestens das von der Sauerstofferzeugungseinheit (50) bereitgestellte Abgas zu befördern, wobei der Gasrückführungskreislauf (502) und der Regenerierungskreislauf (501) Gaskanäle und die Adsorber (130-132) umfassen.

## Claims

1. Installation (50) for supplying respiratory gas to a user (P), comprising:
- a gas source (3) for supplying an oxygen-containing respiratory gas,
- a main gas delivery line (23) fluidically connecting the gas source (3) to a respiratory interface (21) configured to administer the oxygen-containing respiratory gas to the user, during each inspiratory phase of said user,
- a gas recovery line (10) configured to recover and convey at least some of the CO₂/O₂ gas mixture exhaled by the user and located in the respiratory interface, during each expiratory phase of the user, said gas recovery line (10) fluidically connecting the respiratory interface (21) to a gas purification system (1) comprising adsorbers (130-132) arranged in parallel and each containing at least one adsorbent, said gas recovery line (10) supplying said gas purification system (1) with said CO₂/O₂ gas mixture exhaled by the user, and
- a gas recycling line (11) fluidically connecting the gas purification system (1) to the main gas delivery line (23),
**characterized in that** the gas source (3) comprises an oxygen generation unit (30) comprising:
- at least one electrochemical separation module (316),
- a first gas line (3100) for supplying said at least one electrochemical separation module (316) with air,
- gas heating means (313) arranged on the first internal gas line (3100), upstream of said at least one electrochemical separation module (316), and
- a second gas line (3200) fluidically connecting said at least one electrochemical separation module (316) to the gas purification system (1), for conveying a flow of waste gas to the gas purification system (1) in order to regenerate said at least one adsorbent contained in at least one of said adsorbers (130-132).

2. Installation according to Claim 1, **characterized in that** the gas purification system (1) comprises at least three adsorbers (130-132) arranged in parallel.

3. Installation according to Claim 1, **characterized in that** the main gas delivery line (23) comprises a buffer tank (24), and the gas recycling line (11) is fluidically connected to the buffer tank (24).

4. Installation according to Claim 1, **characterized in that** the gas purification system (1) is fluidically connected to the first gas line (3100) of the oxygen generation unit (30) in order to supply air to said first gas line (3100), preferably via a first interconnecting duct (31).

5. Installation according to Claim 4, **characterized in that** the gas purification system (1) comprises an air circuit (500) for conveying air supplied to the first gas line (3100) of the oxygen generation unit (30).

6. Installation according to Claim 5, **characterized in that** the air circuit (500) comprises air passages or ducts (102, 108, 120-122), and at least one adsorber (130-132), preferably it further comprises at least one control valve (110-112; 140-142).

7. Installation according to Claim 1, **characterized in that** said at least one electrochemical separation module (316) of the oxygen generation unit (30) comprises one or more ceramic membranes.

8. Installation according to one of Claims 1, 4 and 5, **characterized in that** the first gas line (3100) of the oxygen generation unit (30) comprises gas suction means (311), said gas heating means (313) being arranged between the gas suction means (311) and said at least one electrochemical separation module (316).

9. Installation according to either of Claims 1 and 8, **characterized in that** heat exchange means (312) are arranged on the first internal gas line (3100), preferably between the gas suction means (311) and the gas heating means (313).

10. Installation according to Claim 1, **characterized in that** the second gas line (3200) conveys a waste gas having an oxygen concentration of less than 21 vol%, typically less than 20 vol%, and/or at a temperature of at least approximately 250°C, preferably at least approximately 300°C.

11. Installation according to Claims 1 and 9, **characterized in that** the second gas line (3200) is arranged in parallel with the first gas line (3100) within said heat exchange means (312) so as to carry out a heat exchange between the air flow circulating in the first gas line (3100) and the waste gas circulating in the second gas line (3200), preferably a counter-current heat exchange.

12. Installation according to either of Claims 1 and 6, **characterized in that**:
- the O₂ generation unit (30) comprises first control means (350, 351) configured to control said at least one electrochemical separation module (316), and/or
- the gas purification system (1) comprises second control means (150, 151) for controlling at least some of the control valves (110-112; 140-142).

13. Installation according to Claim 1, **characterized in that** the respiratory interface (21) is a respiratory mask.

14. Installation according to Claim 1, **characterized in that** a second interconnecting duct (33) fluidically connects the second gas line (3200) of the O₂ generation unit (30) to the gas purification system (1).

15. Installation according to Claim 1, **characterized in that** the gas purification system (1) further comprises:
- a gas recycling circuit (502) for conveying at least the exhaled gas supplied by the gas recovery line (10), and
- a regeneration circuit (501) for conveying at least the waste gas supplied by the oxygen generation unit (50), said gas recycling circuit (502) and regeneration circuit (501) comprising gas ducts and the adsorbers (130-132).
